# EUROPÄISCHE PATENTSCHRIFT

(11) **EP 0 906 311 B1**
(45) Veröffentlichungstag und Bekanntmachung des Hinweises auf die Patenterteilung: **23.04.2003**
(21) Anmeldenummer: 97925028.9
(22) Anmeldetag: 30.05.1997
(51) Int. Cl.: C07D 413/12, C07D 401/12, A01N 43/80, A01N 43/58

(54) **N-SULFONYLIMIDAZOLE ALS FUNGIZIDE**
N-SULFONYLIMIDAZOLES USED AS FUNGICIDES
N-SULFONYLIMIDAZOLES S'UTILISANT COMME FONGICIDES

(30) Priorität: 11.06.1996 DE 19623207
(43) Veröffentlichungstag der Anmeldung: 07.04.1999
(73) Patentinhaber: Bayer CropScience AG, 40789 Monheim (DE)
(72) Erfinder: ASSMANN, Lutz, D-23701 Eutin (DE); ELBE, Hans-Ludwig, D-42329 Wuppertal (DE); TIEMANN, Ralf, D-51375 Leverkusen (DE); HEIL, Markus, D-51381 Leverkusen (DE); STENZEL, Klaus, D-40595 Düsseldorf (DE)
(86) Internationale Anmeldenummer: EP9702830
(87) Internationale Veröffentlichungsnummer: WO97047620

(56) Entgegenhaltungen:
- EP-A- 0 044 394
- EP-A- 0 237 912
- EP-A- 0 238 824
- EP-A- 0 284 277
- EP-A- 0 298 196
- US-A- 3 519 639
- US-A- 3 595 873
- PATENT ABSTRACTS OF JAPAN vol. 11, no. 369 (C-461), 2.Dezember 1987 & JP 62 142164 A (ISHIHARA SANGYO KAISHA LTD.), 25.Juni 1987,
- PATENT ABSTRACTS OF JAPAN vol. 18, no. 19 (C-1152), 13.Januar 1994 & JP 05 255269 A (AGRO KANESHO CO. LTD.), 5.Oktober 1993,

## Beschreibung

Die vorliegende Erfindung betrifft neue Imidazolderivate, mehrere Verfahren zu ihrer Herstellung und deren Verwendung als Mikrobizide im Pflanzenschutz und im Materialschutz.

Es ist bereits bekannt geworden, daß bestimmte Imidazolderivate fungizide Eigenschaften besitzen (vgl. EP-A 0 298 196). So läßt sich z. B. 4-Chlor-2-cyano-1-morpholin-N-ylsulfonyl-5-phenylimidazol oder 4-Chlor-2-cyano-5-phenyl-1-(2-thienylsulfonyl)-imidazol zur Bekämpfung von Pilzen einsetzen. Die Wirksamkeit dieser Stoffe ist gut, läßt aber bei niedrigen Aufwandmengen in manchen Fällen zu wünschen übrig.

Weiterhin sind aus der EP-A 0 284 277, der EP-A 0 044 394, der EP-A 0 238 824, der EP-A 0 237 912, der JP-A 5 - 255 269, der US-A 3 595 873, der US-A 3 519 639 und der JP-A 62 - 142 164 zahlreiche Imidazol - Derivate mit fungiziden Eigenschaften bekannt. In diesen Druckschriften werden aber keine Imidazole erwähnt, in denen ein ungesättigter Heterocyclus, der mindestens ein Stickstoff- oder Sauerstoffatom enthält, über eine SO₂ - Brücke mit einem der beiden Stickstoffatome des Imidazol-Ringes verbunden ist.

Es wurden nun neue Imidazolderivate der Formel 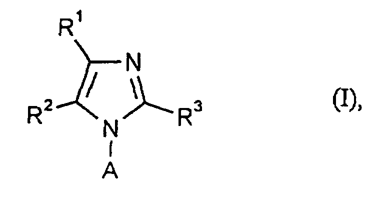 in welcher
- R¹ und R²: gleich oder verschieden sind und unabhängig voneinander für Wasserstoff, Fluor, Chlor, Brom, Iod, Cyano, Thiocyanato, Nitro, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit I bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil, Halogenalkylcarbonyloxy mit 1 bis 4 Kohlenstoffatomen in der Halogenalkylgruppe und 1 bis 5 gleichen oder verschiedenen Halogenatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Alkenyl mit 2 bis 4 Kohlenstoffatomen, Alkenyloxy mit 2 bis 4 Kohlenstoffatomen, Halogenalkylthio mit 1 bis 4 Kohlenstoffatomen und 1 bis 5 gleichen oder verschiedenen Halogenatomen, Halogenalkenyl mit 2 bis 4 Kohlenstoffatomen und 1 bis 5 gleichen oder verschiedenen Halogenatomen, Halogenalkenyloxy mit 2 bis 4 Kohlenstoffatomen und 1 bis 5 gleichen oder verschiedenen Halogenatomen, Alkylsulfonyloxy mit 1 bis 4 Kohlenstoffatomen oder für Z-R⁴ stehen, wobei
- Z: für Alkandiyl mit 1 bis 4 Kohlenstoffatomen, Alkendiyl mit 2 bis 4 Kohlenstoffatomen, Alkindiyl mit 2 bis 4 Kohlenstoffatomen oder eine Gruppe der Formel

-*Q-CQ-, -*CQ-Q-, -*CH₂-Q-, -*Q-CH₂-, -*CQ-Q-CH₂-,

-*CH₂-Q-CQ-, -*Q-CQ-CH₂-, -*Q-CQ-Q-CH₂-, -S(O)ₙ-,

-*CH₂-S(O)ₙ-, -CQ- oder -*S(O)ₙ-CH₂-

steht,
wobei jeweils die mit * gekennzeichneten Atome mit R⁴ verbunden sind,
Q für Sauerstoff oder Schwefel steht,
n für 0, 1 oder 2 steht, und
- R⁴: für Phenyl oder Naphthyl steht, wobei jeder dieser beiden Reste einfach bis dreifach, gleichartig oder verschieden substituiert sein kann durch Halogen, Cyano, Nitro, Formyl, Carbamoyl, Thiocarbamoyl, Alkyl mit 1 bis 4 Kohlenstoffatomen, Alkoxy mit 1 bis 4 Kohlenstoffatomen, Alkylthio mit 1 bis 4 Kohlenstoffatomen, Alkenyl oder Alkenyloxy mit jeweils 2 bis 4 Kohlenstoffatomen, Alkinyl oder Alkinyloxy mit jeweils 2 bis 4 Kohlenstoffatomen, Halogenalkyl, Halogenalkoxy oder Halogenalkylthio mit jeweils 1 bis 4 Kohlenstoffatomen und I bis 5 gleichen oder verschiedenen Halogenatomen, Halogenalkenyl oder Halogenalkenyloxy mit jeweils 2 bis 4 Kohlenstoffatomen und 1 bis 5 gleichen oder verschiedenen Halogenatomen, Halogenalkinyl oder Halogenalkinyloxy mit jeweils 2 bis 4 Kohlenstoffatomen und 1 bis 5 gleichen oder verschiedenen Halogenatomen, Alkylamino, Dialkylamino, Alkoxycarbonyl, Hydroximinoalkyl oder Alkoximinoalkyl mit jeweils 1 bis 4 Kohlenstoffatomen in den einzelnen Alkylteilen, Cycloalkyl mit 3 bis 6 Kohlenstoffatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylcarbonyl mit 1 bis 4 Kohlenstoffatomen in der Alkylgruppe, Alkylcarbonyloxy mit 1 bis 4 Kohlenstoffatomen im Alkylteil und/oder Alkylsulfonyloxy mit 1 bis 4 Kohlenstoffatomen,
oder auch einfach substituiert sein kann durch gegebenenfalls einfach bis vierfach, gleichartig oder verschieden durch Halogen substituiertes, zweifach verknüpftes Dioxyalkylen mit 1 oder 2 Kohlenstoffatomen, wobei die Sauerstoffatome nicht benachbart sind,
oder
- R⁴: für einen ungesättigten Heterocyclyl-Rest mit 5 oder 6 Ringgliedern und 1 bis 3 Heteroatomen, wie Stickstoff, Sauerstoff und/oder Schwefell steht, wobei diese Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoffatomen im Alkoxyteil, Cycloalkyl mit 3 bis 6 Kohlenstoffatomen, Cyano und/oder Nitro,
oder
- Z: für eine direkte Bindung oder für ein Sauerstoffatom steht, wenn
- R⁴: für Phenyl oder Naphthyl steht, wobei jeder dieser beiden Reste einfach bis dreifach, gleichartig oder verschieden substituiert sein kann durch diejenigen Reste, die oben bereits als Phenyl- bzw. Naphthyl-Substituenten genannt wurden,
- R³: für Fluor, Chlor, Brom, Iod, Cyano, Nitro, Trifluor-, methyl, Thiocarbamoyl, Thiocyanato oder die Gruppierung 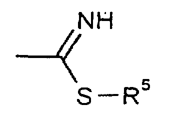 steht,
worin
R⁵ für Alkyl mit 1 bis 4 Kohlenstoffatomen steht,
- A: für eine Gruppierung der Formel -SO₂-R⁶ steht und
- R⁶: für einen ungesättigten Heterocycyl-Rest mit 5 oder 6 Ringgliedem und 1 bis 3 Heteroatomen, wie Sauerstoff, Schwefel und/oder Stickstoff steht, wobei mindestens ein Heteroatom für Stickstoff oder Sauerstoff steht, und wobei die Heterocyclen einfach, zweifach oder auch dreifach gleichartig oder verschieden substituiert sein können durch Halogen, Cyano, Nitro, Hydroxy, Mercapto, Amino, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil, Halogenalkylcarbonyloxy mit 1 bis 4 Kohlenstoffatomen in der Halogenalkylgruppe und 1 bis 5 gleichen oder verschiedenen Halogenatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Halogenalkylthio mit 1 bis 4 Kohlenstoffatomen und 1 bis 5 gleichen oder verschiedenen Halogenatomen, Alkylsulfonyloxy mit 1 bis 4 Kohlenstoffatomen, oder durch jeweils gegebenenfalls einfach oder zweifach durch Methyl substituiertes Pyrrolidinyl, Piperidinyl, Piperidyl, Morpholinyl oder Piperazinyl,
sowie deren Säureadditions-Salze und Metallsalz-Komplexe gefunden.

Weiterhin wurde gefunden, daß man Imidazolderivate der Formel (I) sowie deren Säureadditions-Salze und Metallsalz-Komplexe erhält, wenn man
a) Imidazole der Formel 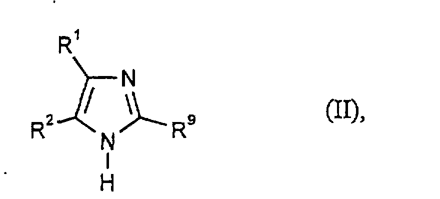 in welcher
   - R¹ und R²: die oben angegebenen Bedeutungen haben und
   - R⁹: für Fluor, Chlor, Brom, Cyano, Nitro, Trifluormethyl oder Thiocyanato steht,
   mit Halogeniden der Formel

   A-X¹ (III)

   in welcher
   - A: die oben angegebene Bedeutung hat und
   - X¹: für Halogen steht,
   gegebenenfalls in Gegenwart eines Säurebindemittels und gegebenenfalls in Gegenwart eines Verdünnungsmittels umsetzt,
   oder wenn man
b) Cyanimidazole der Formel 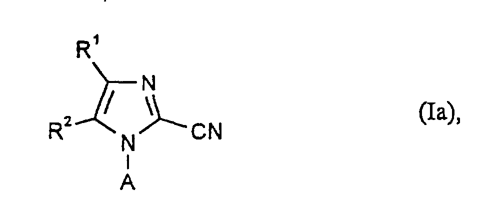 in welcher
   - R¹, R² und A: die oben angegebenen Bedeutungen haben,
   mit Schwefelwasserstoff, gegebenenfalls in Gegenwart einer Base und gegebenenfalls in Gegenwart eines Verdünnungsmittels umsetzt, oder wenn man
c) Thiocarbamoylimidazole der Formel 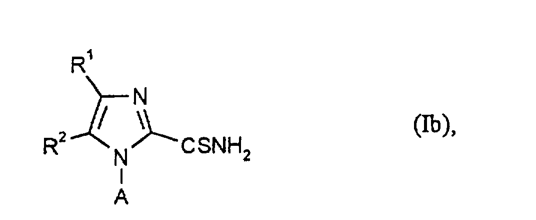 in welcher
   - R¹, R² und A: die oben angegebenen Bedeutungen haben,
   mit Alkylierungsmitteln der Formel

   R⁵-X² (IV)

   in welcher
   - R⁵: die oben angegebene Bedeutung hat und
   - X²: für eine Abgangsgruppe steht,
   gegebenenfalls in Gegenwart eines Säurebindemittels und gegebenenfalls in Gegenwart eines Verdünnungsmittels umsetzt,
   und gegebenenfalls an die so erhaltenen Verbindungen der Formel (I) eine Säure oder ein Metallsalz addiert.

Schließlich wurde gerunden, daß die Imidazolderivate der Formel (I) sowie deren Säureadditions-Salze und Metallsalz-Komplexe sehr gute mikrobizide Eigenschaften besitzen und sowohl im Pflanzenschutz als auch im Materialschutz zur Bekämpfung unerwünschter Mikroorganismen eingesetzt werden können.

Überraschenderweise zeigen die erfindungsgemäßen Stoffe eine bessere fungizide Wirkung als 4-Chlor-2-cyano-1-morpholin-N-ylsulfonyl-5-phenylimidazol oder 4-Chlor-2-cyano-5-phenyl-1-(2-thienylsulfonyl)-imidazol, welche konstitutionell ähnliche, vorbekannte Wirkstoffe gleicher Wirkungsrichtung sind.

Die erfindungsgemäßen Stoffe sind durch die Formel (I) allgemein definiert.
- R¹ und R²: stehen bevorzugt unabhängig voneinander für Wasserstoff, Fluor, Chlor, Brom, Iod, Cyano, Thiocyanato, Nitro, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n-oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroxyiminomethyl, Hydroxyiminoethyl, Methoxyiminomethyl, Ethoxyiminomethyl, Methoxyiminoethyl und/oder Ethoxyiminoethyl oder für Z-R⁴.
Z steht bevorzugt für Methandiyl, 1,1-Ethandiyl, 1,2-Ethandiyl, 1,1-, 1,2-, 1,3- oder 2,2-Propandiyl, 1,1-Ethendiyl, 1,2-Ethendiyl, Ethindiyl,

-*Q-CQ-, -*CQ-Q-, -*CH₂-Q-, -*Q-CH₂-, -*CQ-Q-CH₂-,

-*CH₂-Q-CQ-, -*Q-CQ-CH₂-, -*Q-CQ-Q-CH₂-, -S(O)ₙ-,

-*CH₂-S(O)ₙ-, -CQ- oder -*S(O)ₙ-CH₂-,

wobei jeweils die mit * gekennzeichneten Atome mit R⁴ verbunden sind.
Q steht auch bevorzugt für Sauerstoff oder Schwefel.
n steht auch bevorzugt für 0, 1 oder 2.
R⁴ steht bevorzugt für Phenyl oder Naphthyl, wobei jeder dieser Reste einfach bis dreifach, gleichartig oder verschieden substituiert sein kann durch Fluor, Chlor, Brom, Cyano, Nitro, Formyl, Carbamoyl, Thiocarbamoyl, Methyl, Ethyl, n- oder i-Propyl, n-, i-, s- oder t-Butyl, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, Cyclopropyl, Cyclobutyl, Cyclopentyl und/oder Cyclohexyl,
oder auch einfach substituiert sein kann durch jeweils gegebenenfalls einfach bis vierfach durch Fluor und/oder Chlor substituiertes jeweils zweifach verknüpftes Methylendioxy oder Ethylendioxy.
R⁴ steht weiterhin bevorzugt für einen ungesättigten Heterocyclyl-Rest mit 5 oder 6 Ringgliedern und 1 bis 3 Heteroatomen, wie Stickstoff, Sauerstoff und/oder Schwefel, wobei jeder dieser Reste einfach oder zweifach, gleichartig oder verschieden substituiert sein kann durch Fluor, Chlor, Brom, Methyl, Ethyl, Methoxy, Ethoxy, Halogenalkyl mit 1 oder 2 Kohlenstoffatomen und 1 bis 5 Fluor-, Chlor- und/oder Bromatomen, Halogenalkoxy mit 1 oder 2 Kohlenstoffatomen und 1 bis 5 Fluor-, Chlorund/oder Bromatomen, Methoxycarbonyl, Ethoxycarbonyl, Cycloalkyl mit 3 bis 6 Kohlenstoffatomen, Cyano und/oder Nitro.
- Z: steht außerdem auch bevorzugt für eine direkte Bindung oder für ein Sauerstoffatom, wenn
- R⁴: für Phenyl oder Naphthyl steht, wobei jeder dieser Reste einfach bis dreifach, gleichartig oder verschieden substituiert sein kann durch diejenigen Reste, die oben bereits als besonders bevorzugte Substituenten am Phenyl- bzw. Naphthyl-Rest genannt wurden.
- R³: steht bevorzugt für Fluor, Chlor, Brom, Iod, Cyano, Nitro, Trifluormethyl, Thiocarbamoyl, Thiocyanato oder die Gruppierung 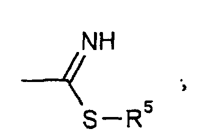 worin
R⁵ für Methyl oder Ethyl steht.
- A: steht auch bevorzugt für eine Gruppierung der Formel -SO₂-R⁶.
- R⁶: steht bevorzugt für Pyrrolyl, Furyl, Pyrazolyl, Imidazolyl, Thiazolyl, Isothiazolyl, Oxazolyl, Isoxazolyl, Oxadiazolyl, Thiadiazolyl, 1,2,3-Triazinyl, 1,2,4-Triazinyl, 1,3,5-Triazinyl, Pyridyl, Pyrimidyl, Pyrazinyl oder Pyridazinyl, wobei diese Reste einfach, zweifach oder dreifach gleichartig oder verschieden substituiert sein können durch Fluor, Chlor, Brom, Cyano, Nitro, Amino, Hydroxy, Mercapto, Formyl, Carboxy, Carbamoyl, Thiocarbamoyl, Methyl, Ethyl, n- oder i-Propyl, n-, i-, s- oder t-Butyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroxyiminomethyl, Hydroxyiminoethyl, Methoxyiminomethyl, Ethoxyiminomethyl, Methoxyiminoethyl, Ethoxyiminoethyl, oder durch jeweils gegebenenfalls einfach oder zweifach durch Methyl substituiertes Pyrrolidinyl, Piperidyl, Morpholinyl oder Piperazinyl.

Bevorzugte erfindungsgemäße Verbindungen sind auch Additionsprodukte aus Säuren und denjenigen Imidazolderivaten der Formel (I), in denen R¹, R², R³ und A diejenigen Bedeutungen haben, die für diese Reste als bevorzugt genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure, Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Imidazolderivaten der Formel (I), in denen R¹, R², R³ und A diejenigen Bedeutungen haben, die für diese Reste als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in den folgenden Tabellen 1 bis 13 aufgeführten Imidazolderivate genannt:

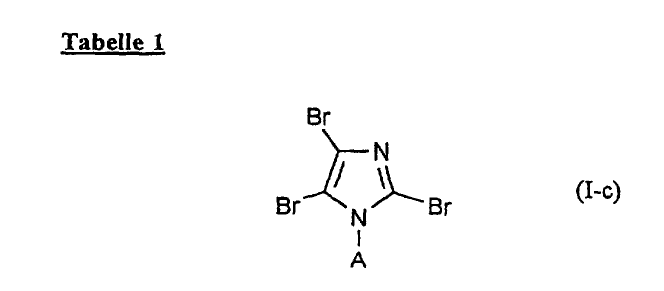

wobei A für die folgenden Substituenten steht:

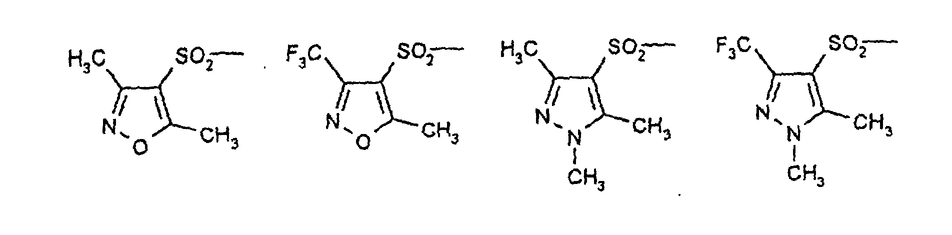

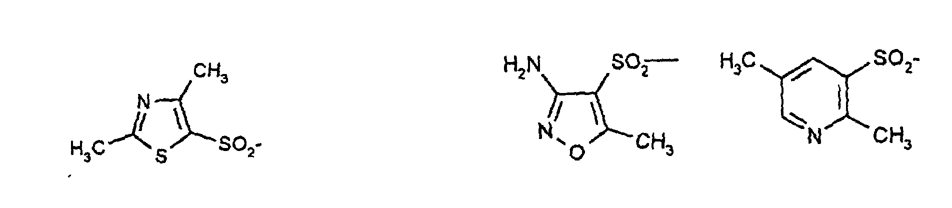

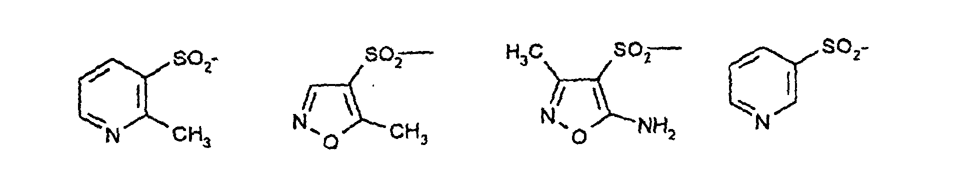

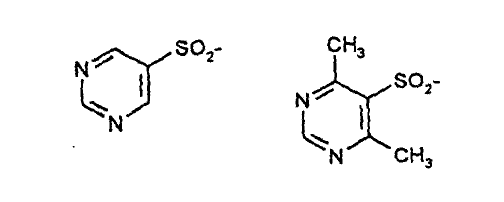

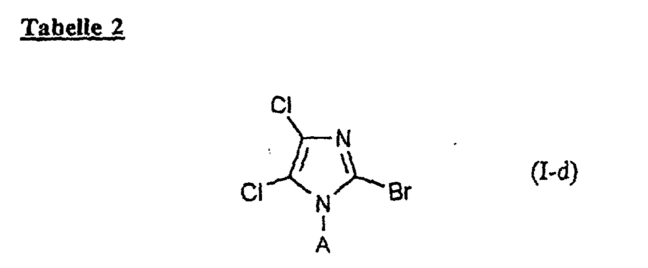

wobei A für die in Tabelle 1 genannten Substituenten steht.

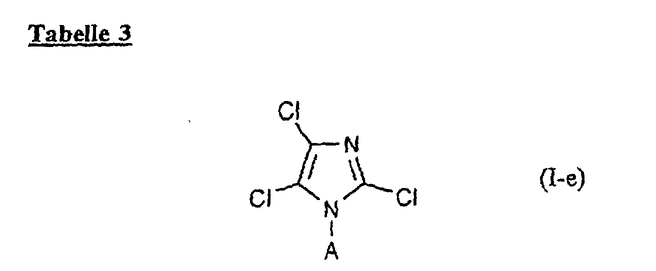

wobei A für die in Tabelle 1 genannten Substituenten steht.

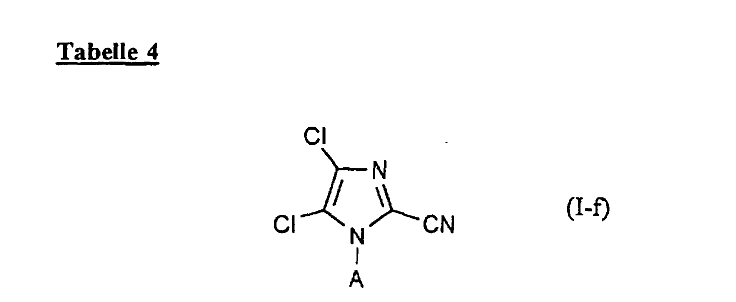

wobei A für die in Tabelle 1 genannten Substituenten steht.

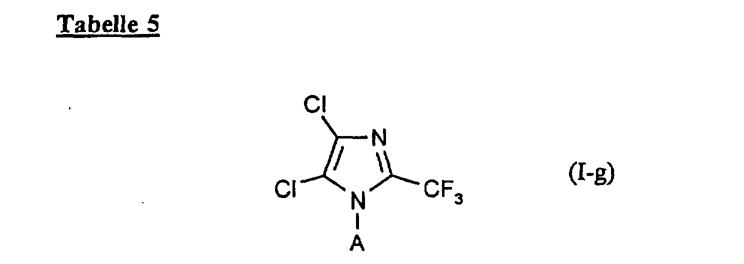

wobei A für die in Tabelle 1 genannten Substituenten steht.

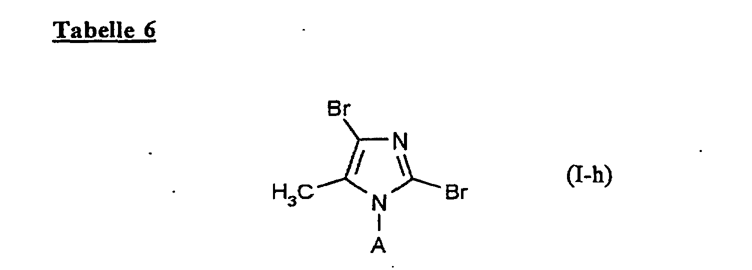

wobei A für die in Tabelle 1 genannten Substituenten steht.

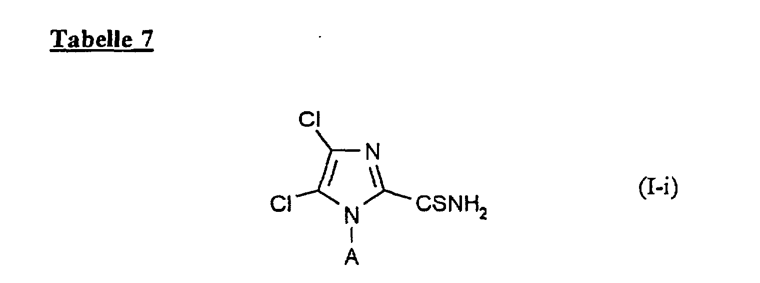

wobei A für die in Tabelle 1 genannten Substituenten steht.

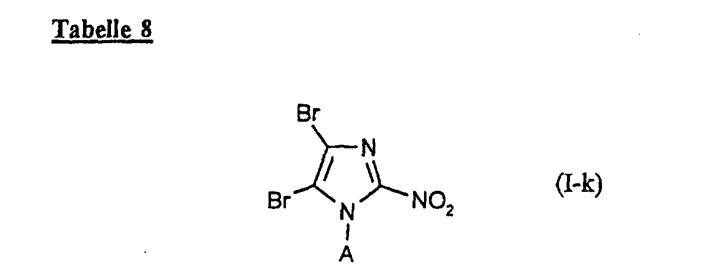

wobei A für die in Tabelle 1 genannten Substituenten steht.

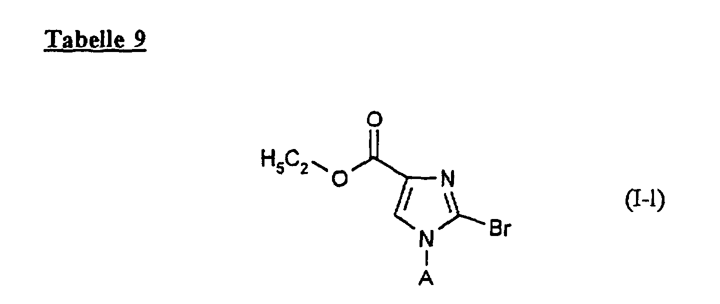

wobei A für die in Tabelle 1 genannten Substituenten steht.

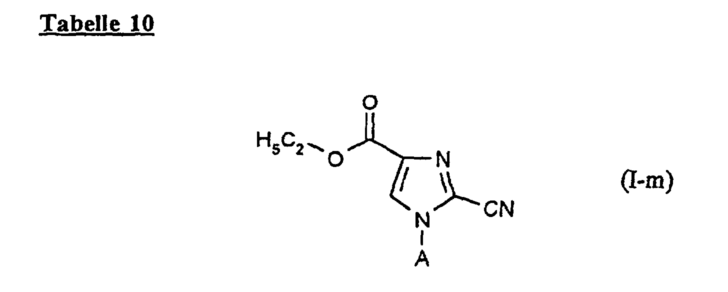

wobei A für die in Tabelle 1 genannten Substituenten steht.

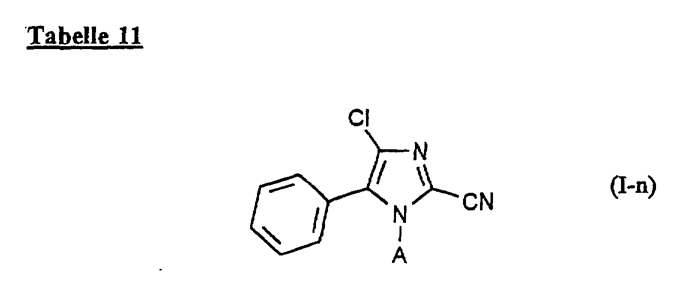

wobei A für die in Tabelle 1 genannten Substituenten steht.

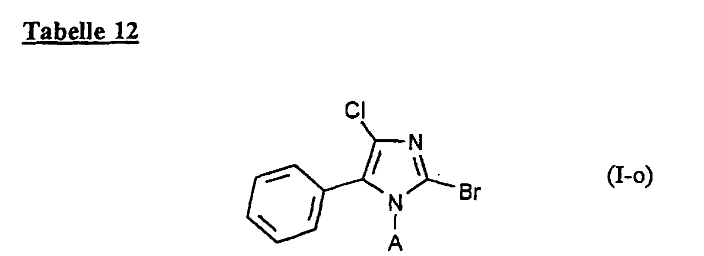

wobei A für die in Tabelle 1 genannten Substituenten steht.

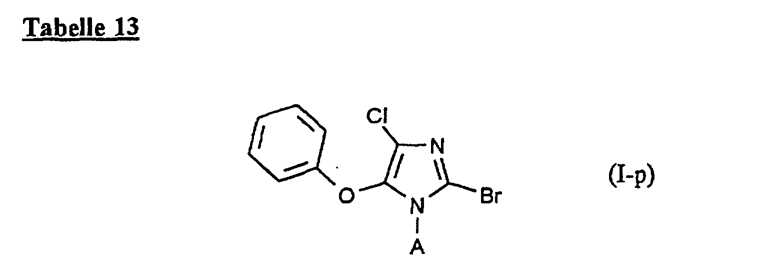

wobei A für die in Tabelle 1 genannten Substituenten steht.

Verwendet man 2,4,5-Tribromimidazol und Furan-2-sulfonylchlorid als Ausgangsstoffe, so läßt sich der Verlauf der Stufe (a) des erfindungsgemäßen Verfahrens durch das folgende Formelschema veranschaulichen:

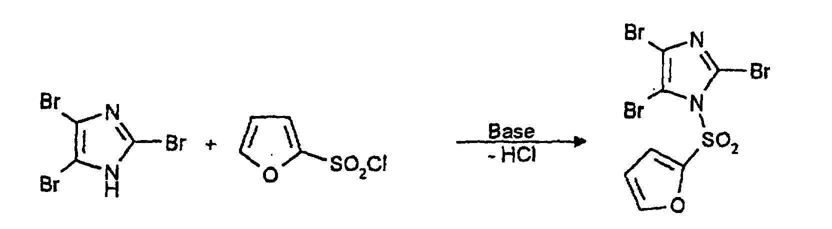

Verwendet man 4-Chlor-2-cyan-1-(2-furyl-sulfonyl)-5-phenyl-imidazol als Ausgangsstoff und Schwefelwasserstoff als Reaktionskomponente, so kann der Verlauf der Stufe (b) des erfindungsgemäßen Verfahrens durch das folgende Formelschema veranschaulicht werden:

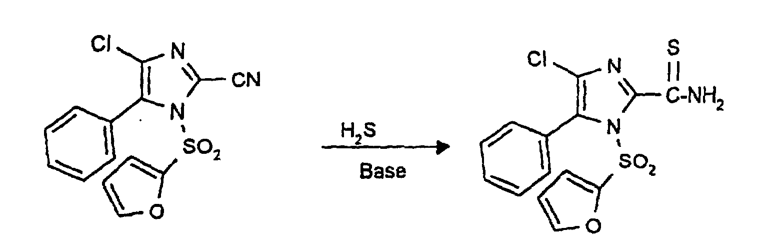

Verwendet man 4-Chlor-1-(2-fürylsulfonyl)-5-phenyl-imidazol-2-thiocarbamid als Ausgangsstoff und Iodmethan als Reaktionskomponente, so kann der Verlauf der Stufe (c) des erfindungsgemäßen Verfahrens durch das folgende Formelschema veranschaulicht werden:

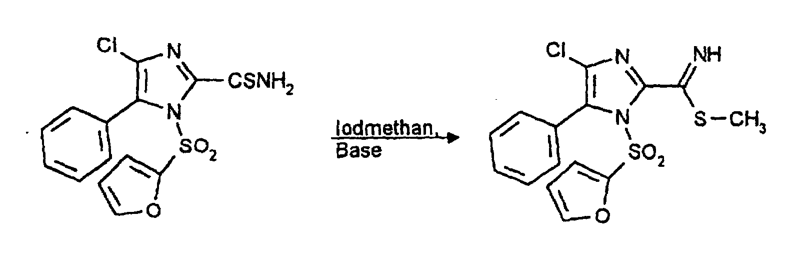

Die bei der Durchführung der Stufe (a) des erfindungsgemäßen Verfahrens als Ausgangsstoffe benötigten Imidazole sind durch die Formel (II) allgemein definiert. In dieser Formel haben R¹ und R² vorzugsweise diejenigen Bedeutungen, die bereits im Zusammenhang mit der Beschreibung der eifindungsgemäßen Stoffe der Formel (I) vorzugsweise für diese Reste genannt wurden.

Die Imidazole der Formel (II) sind bekannt oder lassen sich nach bekannten Methoden herstellen (vgl. Chem. Ber. (1877) 10, 1370; J.Heterocycl.Chem. (1967) 4, 399; Chem.Ber. (1976) 109, 1625; DE-A 2634053; DE-A 2646192; J. Chem. Soc. (1910) 97, 1824; J. Chem. Soc. Perkin Trans. 1 (1989) 95-99; ACS Symp. Ser. (1995), 584 (Synthesis and Chemistry of Agrochemicals IV), 375-83; Tetrahedron (1994), 50(19), 5741-52; EP-A 0390506; Synlett (1990), (5), 277-8; WO 95-17390; EP-A 0298196).

Die bei der Durchführung der Stufe (a) des erfindungsgemäßen Verfahrens als Reaktionskomponenten benötigten Halogenide sind durch die Formel (III) allgemein definiert. In dieser Formel hat A vorzugsweise diejenigen Bedeutungen, die bereits im Zusammenhang mit der Beschreibung der erfindungsgemäßen Stoffe der Formel (I) vorzugsweise für diesen Substituenten genannt wurden.

X¹ steht vorzugsweise für Chlor oder Brom.

Die Halogenide der Formel (III) sind bekannt oder lassen sich nach bekannten Methoden herstellen (vgl. J. Heterocyclic Chem. 1981, 997-1006).

Bei der Durchführung der Stufe (a) des erfindungsgemäßen Verfahrens arbeitet man vorzugsweise in Gegenwart eines Säurebindemittels. Als solche kommen alle üblichen anorganischen und organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner Ammonium-Verbindungen, wie Ammoniumhydroxid, Ammoniumacetat oder Ammoniumcarbonat, und außerdem tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der Stufe (a) des erfindungsgemäßen Verfahrens alle üblichen inerten, organischen Solventien in Betracht. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan, oder Amine, wie Pyridin.

Die Reaktionstemperaturen können bei der Durchführung der Stufe (a) des erfindungsgemäßen Verfahrens in einem größeren Bereich variiert werden. Im allgemeinen arbeitet man bei Temperaturen zwischen 0 und 150°C, vorzugsweise bei Temperaturen zwischen 20 und 120°C.

Sowohl bei der Durchführung der Stufe (a) als auch der Stufen (b) und (c) des erfindungsgemäßen Verfahrens arbeitet man im allgemeinen unter Atmosphärendruck. Es ist jedoch auch möglich, unter erhöhtem oder vermindertem Druck zu arbeiten.

Bei der Durchführung der Stufe (a) des erfindungsgemäßen Verfahrens setzt man auf 1 Mol an Imidazol der Formel (II) im allgemeinen 1 bis 15 Mol, vorzugsweise 1 bis 2 Mol, besonders bevorzugt 1 bis 1,3 Mol an Halogenid der Formel (III) ein. Die Aufarbeitung erfolgt nach üblichen Methoden. Im allgemeinen verfährt man in der Weise, daß man das Reaktionsgemisch mit Wasser verdünnt, danach mit einem mit Wasser wenig mischbaren organischen Lösungsmittel extrahiert, die vereinigten organischen Phasen trocknet und unter vermindertem Druck einengt. Das dabei verbleibende Produkt kann nach üblichen Methoden, wie Chromatographie oder Umkristallisation, von eventuell noch vorhandenen Verunreinigungen befreit werden. Wird die Umsetzung in einem mit Wasser mischbaren organischen Solvens durchgeführt, so fällt das gewünschte Produkt beim Verdünnen des Reaktionsgemisches mit Wasser manchmal als Feststoff an. In diesen Fällen erübrigt sich eine Extraktion. Man trennt vielmehr den Feststoff ab und reinigt ihn gegebenenfalls nach üblichen Methoden.

Die bei der Durchführung der Stufe (b) des erfindungsgemäßen Verfahrens als Ausgangsstoffe benötigten Cyanimidazole sind durch die Formel (Ia) allgemein definiert. Es handelt sich hierbei um erfindungsgemäße Stoffe, die durch Umsetzungen nach Stufe (a) des erfindungsgemäßen Verfahrens herstellbar sind.

Als Basen kommen bei der Durchführung der Stufe (b) des erfindungsgemäßen Verfahrens alle üblichen anorganischen und organischen Säurebindemittel in Frage. Vorzugsweise verwendbar sind diejenigen Säureakzeptoren, die bereits im Zusammenhang mit der Beschreibung der Stufe (a) des erfindungsgemäßen Verfahrens als bevorzugte Säurebindemittel genannt wurden.

Als Verdünnungsmittel kommen bei der Durchführung des Stufe (b) des erfindungsgemäßen Verfahrens Wasser sowie alle üblichen inerten, organischen Solventien in Betracht. Vorzugsweise verwendbar sind gegebenenfalls halogenierte aliphatische, alicyclische oder aromatische Kohlenwasserstoffe, wie beispielsweise Petrolether, Hexan, Heptan, Cyclohexan, Methylcyclohexan, Benzol, Toluol, Xylol, Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Amine, wie Pyridin; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können auch bei der Durchführung der Stufe (b) des erfindungsgemäßen Verfahrens in einem größeren Bereich variiert werden. Im allgemeinen arbeitet man bei Temperaturen zwischen 0°C und 150°C, vorzugsweise zwischen 20°C und 120°C.

Bei der Durchführung der Stufe (b) des erfindungsgemäßen Verfahrens setzt man auf 1 Mol an Cyanimidazol der Formel (Ia) im allgemeinen 1 bis 1000 Mol, vorzugsweise 1 bis 50 Mol an Schwefelwasserstoff ein. Die Aufarbeitung erfolgt nach üblichen Methoden. Im allgemeinen verfährt man in der Weise, daß man das Reaktionsgemisch mit Wasser verdünnt, dann mit einem mit wenig mischbaren organischen Solvens extrahiert, die vereinigten organischen Phasen trocknet und unter vermindertem Druck einengt. Das verbleibende Produkt kann nach üblichen Methoden, wie Chromatographie oder Umkristallisation, von eventuell noch vorhandenen Verunreinigungen befreit werden.

Die bei der Durchführung der Stufe (c) des erfindungsgemäßen Verfahrens als Ausgangsstoffe benötigten Thiocarbamoylimidazole sind durch die Formel (Ib) allgemein definiert. Es handelt sich hierbei um erfindungsgemäße Stoffe, die durch Umsetzungen nach Stufe (b) des erfindungsgemäßen Verfahrens herstellbar sind.

Die bei der Durchführung der Stufe (c) des erfindungsgemäßen Verfahrens als Reaktionskomponenten benötigten Alkylierungsmittel sind durch die Formel (IV) allgemein definiert. In dieser Formel hat R⁵ vorzugsweise diejenigen Bedeutungen, die bereits im Zusammenhang mit der Beschreibung der erfindungsgemäßen Stoffe der Formel (I) für diesen Rest als bevorzugt genannt wurden. X² steht vorzugsweise für Chlor, Brom, Iod, Methylsulfonyloxy, Tolylsulfonyloxy oder einen Rest der Formel R⁵-O-SO₂-O- oder R⁵-O-CO-O-, worin R⁵ die oben vorzugsweise genannten Bedeutungen hat.

Als Säurebindemittel kommen bei der Durchführung der Stufe (c) des erfindungsgemäßen Verfahrens alle üblichen anorganischen und organischen Basen in Frage. Vorzugsweise verwendbar sind diejenigen Säureakzeptoren, die bereits im Zusammenhang mit der Beschreibung der Stufe (a) des erfindungsgemäßen Verfahrens als bevorzugte Säurebindemittel genannt wurden.

Als Verdünnungsmittel kommen bei der Durchführung der Stufe (c) des erfindungsgemäßen Verfahrens alle üblichen inerten, organischen Solventien in Betracht. Vorzugsweise verwendbar sind diejenigen Verdünnungsmittel, die bereits im Zusammenhang mit der Stufe (a) des erfindungsgemäßen Verfahrens als bevorzugt verwendbare Solventien genannt wurden.

Die Reaktionstemperaturen können auch bei der Durchführung der Stufe (c) des erfindungsgemäßen Verfahrens in einem größeren Bereich variiert werden. Im allgemeinen arbeitet man bei Temperaturen zwischen 0°C und 150°C, vorzugsweise zwischen 20!C und 120°C.

Bei der Durchführung der Stufe (c) des erfindungsgemäßen Verfahrens setzt man auf 1 Mol an Thiocarbamoylimidazol der Formel (Ib) im allgemeinen 1 bis 10 Mol, vorzugsweise 1 bis 5 Mol an Alkylierungsmittel der Formel (IV) ein. Die Aufarbeitung erfolgt wiederum nach üblichen Methoden.

Die Imidazolderivate der Formel (I) können in Säureadditions-Salze oder Metallsalz-Komplexe überführt werden.

Zur Herstellung Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia-, Podosphaera-, Phytophtora- und Plasmopara-Arten, einsetzen. Mit gutem Erfolg werden auch Reiskrankheiten, wie beispielsweise Pyricularia-Arten, bekämpft.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Allzarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
   Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilate, Iminoctadinetriacetate, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   2',6'-Dibrom-2-methyl-4'-trifluormethoxy-4'-trifluor-methyl-1,3-thiazol-5-carboxanilid,
   2,6-Dichlor-N-(4-trifluormethylbenzyl)-benzamid,
   2-Aminobutan,
   2-Phenylphenol(OPP),
   8-Hydroxychinolinsulfat,
   cis-1-(4-Chlorphenyl)-2-(1H-1,2,4-triazol-1-yl)-cycloheptanol,
   (5RS,6RS)-6-Hydroxy-2,2,7,7-tetramethyl-5-(1H-1,2,4-triazol-1-yl)-3-octanon,
   α-(2,4-Dichlorphenyl)-βmethoxy-α-methyl-1H-1,2,4-triazol-1-ethanol,
   α-(1,1-Dimethylethyl)-β-(2-phenoxyethyl)-1H-1,2,4-triazol-1-ethanol,
   1-[1-[2-[(2,4-Dichlorphenyl)-methoxy]-phenyl]-ethenyl]-1H-imidazol,
   bis-(1-Methylethyl)-3-methyl-4-[(3-methylbenzoyl)-oxy]-2,5-thiophendicarboxylat,
   2,6-Dichlor-N-[[4-(trifluormethyl)-phenyl]-methyl]-benzamid,
   (E)-α-(Methoxyimino)-N-methyl-2-phenoxy-phenylacetamid,
   9H-Xanthen-9-carbonsäure-2-[(phenylamino)-carbonyl]-hydrazid,
   O-Methyl-S-phenyl-phenylpropylphosphoramidothioate,
   N-(5-Chlor-2-methylphenyl)-2-methoxy-N-(2-oxo-3-oxazolidinyl)-acetamid,
   1-(2,4-Dichlorphenyl)-2-(1H-1,2,4-triazol-1-yl)ethanon-O-(phenylmethyl)-oxim,
   N-(2,6-Dimethylphenyl)-2-methoxy-N-(tetrahydro-2-oxo-3-thienyl)-acetamid,
   cis-4-[3-[4-(1,1-Dimethylpropyl)-phenyl-2-methylpropyl]-2,6-dimethyl-morpholinhydrochlorid,
   1-(3,5-Dichlorphenyl)-3-(2-propenyl)-2,5-pyrrolidindion,
   1-Methyl-5-nonyl-2-(phenylmethyl)-3-pyrrolidinol,
   1-[[2-(4-Chlorphenyl)-3-phenyloxiranyl]-methyl]-1H-1,2,4-triazol,
   Methantetrathiol-Natriumsalz,
   2-(2,3,3-Triiod-2-propenyl)-2H-tetrazol,
   N-[3-Chlor-4,5-bis(2-propinyloxy)-phenyl]-N'-methoxy-methanimidamid,
   α-(5-Methyl-1,3-dioxan-5-yl)-β-[[4-(trifluormethyl)-phenyl]-methylen]-1H-1,2,4-triazol-1-ethanol,
   1-(2-Methyl-1-naphthalenyl)-1H-pyrrol-2,5-dion,
   N-(2,6-Dimethylphenyl)-2-methoxy-N-(tetrahydro-2-oxo-3-furanyl)-acetamid,
   3,4-Dichlor-1-[4-(difluormethoxy)-phenyl]-1H-pyrrol-2,5-dion,
   N-[2,2,2-Trichlor-1-[(chloracetyl)-amino]-ethyl]-benzamid,
   N-Formyl-N-hydroxy-DL-alanin-Natriumsalz,
   N-(4-Cyclohexylphenyl)-1,4,5,6-tetrahydro-2-pyrimidinamin,
   4-Methyl-tetrazolo[1,5-a]quinazolin-5(4H)-on,
   2-Chlor-N-(2,6-dimethylphenyl)-N-(isothiocyanatomethyl)-acetamid,
   Ethyl-[(4-chlorphenyl)-azo]-cyanoacetat,
   N-(4-Hexylphenyl)-1,4,5,6-tetrahydro-2-pyrimidinamin,
   N-(2-Chlor-4-nitrophenyl)-4-methyl-3-nitro-benzolsulfonamid,
   Methyl-N-(chloracetyl)-N-(2,6-dimethylphenyl)-DL-alaninat,
   3-[2-(4-Chlorphenyl)-5-ethoxy-3-isoxazolidinyl]-pyridin,
   2-[(1-Methylethyl)sulfonyl]-5-(trichlormethyl)-1,3,4-thiadiazol,
   spiro[2H]-1-Benzopyran-2,1'(3'H)-isobenzofuran]-3'-on,
   Methyl-N-(2,6-dimethylphenyl)-N-(5-isoxazolylcarbonyl)-DL-alaninat,
   Kaliumhydrogencarbonat,
   1-[[2-(2,4-Dichlorphenyl)-1,3-dioxolan-2-yl]-methyl]-1H-imidazol,
   1-[(Diiodmethyl)-sulfonyl]-4-methyl-benzol,
   2-Brom-2-(brommethyl)-pentandinitril,
   2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
   Methyl-1-(2,3-dihydro-2,2-dimethyl-1H-inden-1-yl)-1H-imidazol-5-carboxylat,
   2-Chlor-N-(2,3-dihydro-1,1,3-trimethyl-1H-inden-4-yl)-3-pyridincarboxamid,
   O,O-Diethyl-[2-(dipropylamino)-2-oxoethyl]-ethylphosphoramidothioat,
   α-(2,4-Dichlorphenyl)-β-fluor-β-propyl-1H-1,2,4-triazol-1-ethanol,
   3-(1,1-Dimethylpropyl-1-oxo-1H-inden-2-carbonitril,
   2,6-Dichlor-5-(methylthio)-4-pyrimidinyl-thiocyanat,
   S-Methyl-1,2,3-benzothiadiazol-7-carbothioat,
   N-(6-Methoxy)-3-pyridinyl)-cyclopropancarboxamid,
   3,5-Dichlor-N-[cyan[(1-methyl-2-propynyl)-oxy]-methyl]-benzamid,
   4-Chlor-2-cyan-N,N-dimethyl-5-(4-methylphenyl)-1H-imidazol-1-sulfonamid,
   8-(1,1-Dimethylethyl)-N-ethyl-N-propyl-1,4-dioxaspiro[4.5]decan-2-methanamin,
   2,2-Dichlor-N-[1-(4-chlorphenyl)ethyl]-1-ethyl-3-methyl-cyclopropancarboxamid,
   N-(2,3-Dichlor-4-hydroxyphenyl)-1-methyl-cyclohexancarboxamid.
**Bakterizide:**
   Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe besitzen in manchen Fällen auch herbizide Eigenschaften.

Die Herstellung und die Verwendung von erfindungsgemäßen Wirkstoffen werden durch die folgenden Beispiele veranschaulicht.

### Herstellungsbeispiele:

### Beispiel 1

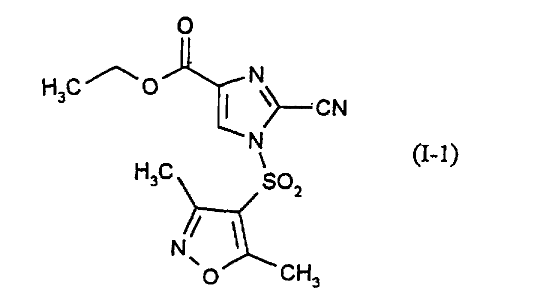

Eine Lösung von 2,5 g (15 mmol) 2-Cyanimidazol-4-carbonsäureethylester in 40 ml absolutem Tetrahdrofuran wird mit 0,6 g (15 mmol) Natriumhydrid (60%ige Mineralölsuspension) versetzt und 10 Minuten bei 20°C gerührt. Anschließend gibt man 3,0 g (15 mmol) 3,5-Dimethylisoxazol-4-sulfonylchlorid zu und rührt weitere 20 Stunden bei Raumtemperatur. Danach wird das Reaktionsgemisch mit 150 ml Wasser verdünnt. Das entstehende Gemisch wird mehrfach mit je 150 ml Essigsäureethylester extrahiert. Die vereinigten organischen Phasen werden nach dem Trocknen über Natriumsulfat unter vermindertem Druck eingeengt. Der verbleibende Rückstand wird mit Dichlormethan als Laufmittel an Kieselgel chromatographiert. Nach dem Einengen des Eluates unter vermindertem Druck erhält man 1,4 g (32 % der Theorie) an 2-Cyan-1-(3,5-dimethyl-isoxazol-4-ylsulfonyl)-imidazol-4-carbonsäure-ethylester in Form eines farblosen Feststoffes vom Schmelzpunkt 150 bis 153°C.

### Beispiel 2

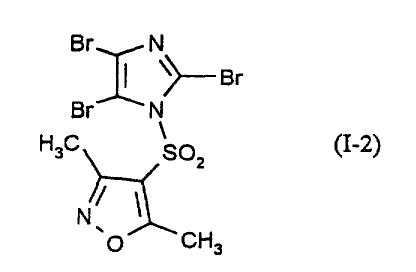

Zu einer Lösung von 3.0 g (5 mmol) 2,4,5-Tribromimidazol in 30 ml Acetonitril gibt man 2,0 g (15 mmol) Kaliumcarbonat und rührt 10 Minuten bei 20°C. Danach versetzt man mit 1,9 g (10 mmol) 3,5-Dimethylisoxazol-4-sulfonylchlorid und rührt 20 Stunden bei 20°C. Anschließend wird die Reaktionsmischung auf 100 ml Wasser gegeben. Der erhaltene Niederschlag abfiltriert und getrocknet.

Man erhält 2,7 g (58% der Theorie) an 1-(3,5-Dimethylisoxazol-4-yl-sulfonyl)-2,4,5-tribromimidazol in Form eines gelben Feststoffes vom Schmelzpunkt 165 bis 170°C.

### Beispiel 3

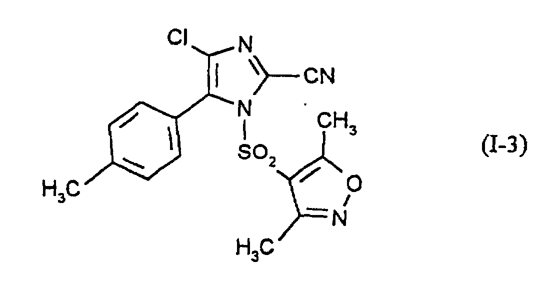

Zu einer Lösung von 2,2 g (10 mmol) 4-Chlor-5-(4-tolyl)-imidazol-2-carbonitril in 30 ml Acetonitril gibt man 2,0 g (15 mmol) Kaliumcarbonat und rührt 10 Minuten bei 20°C. Danach versetzt man mit 1,9 g (10 mmol) 3,5-Dimethylisoxazol-4-sulfonylchlorid und rührt 4 Stunden bei 20°C. Anschließend wird die Reaktionsmischung auf 150 ml Wasser gegeben und mit 50 ml Essigsäureethylester extrahiert. Die vereinigten organischen Phasen werden über Natriumsulfat getrocknet und danach unter vermindertem Druck eingeengt. Der verbleibende Rückstand wird mit Methylenchlorid als Laufmittel an Kieselgel chromatographiert. Durch Einengen des Eluates unter vermindertem Druck erhält man 1,8 g (50 % der Theorie) an 4-Chlor-1-(3,5-dimethyl-isoxazol-4-yl-sulfonyl)-5-(4-tolyl)-imidazol-2-carbonitril in Form eines farblosen Feststoffes vom Schmelzpunkt 103 bis 108°C.

Nach den zuvor angegebenen Methoden werden auch die in der folgenden Tabelle 14 aufgeführten erfindungsgemäßen Stoffe hergestellt.

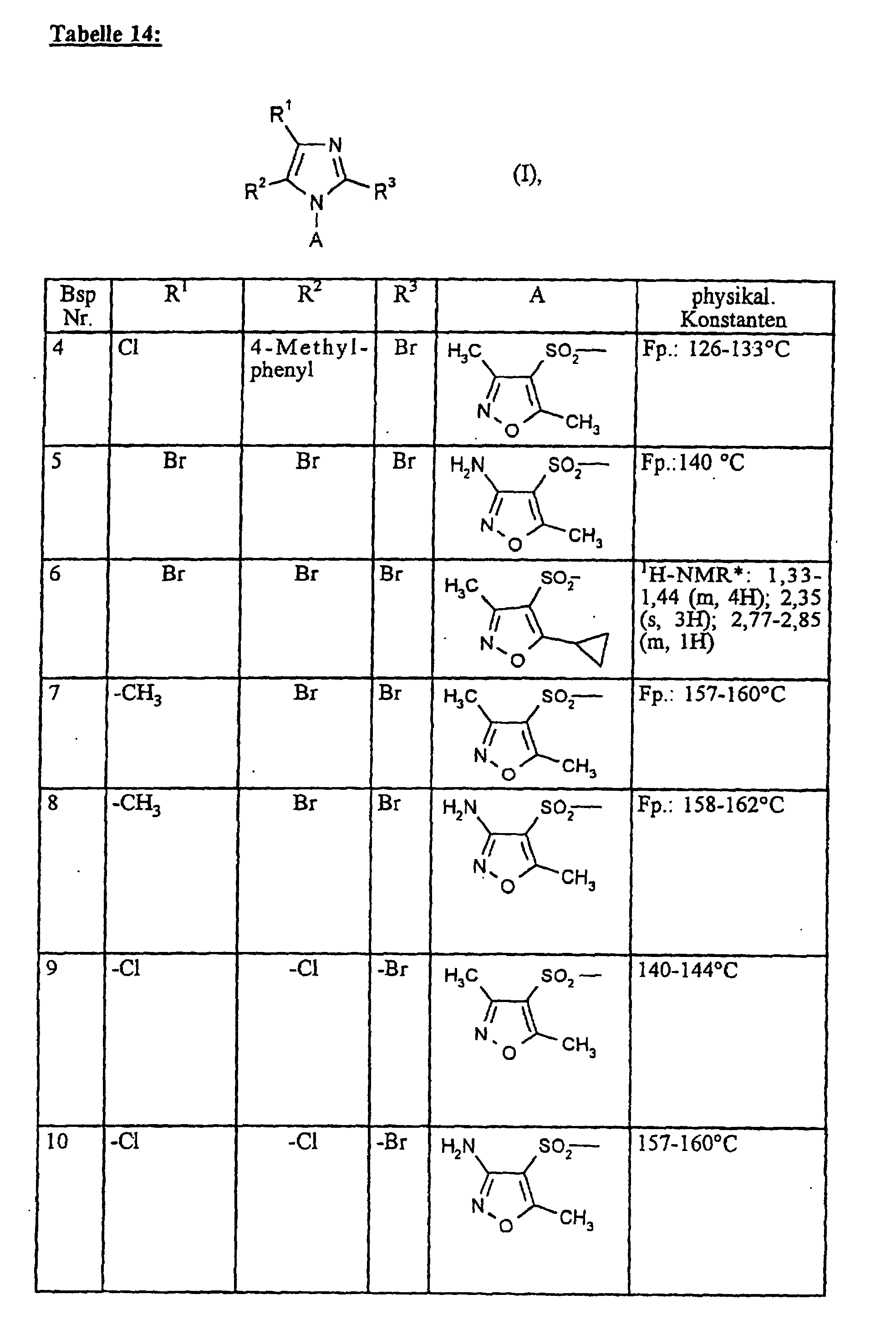

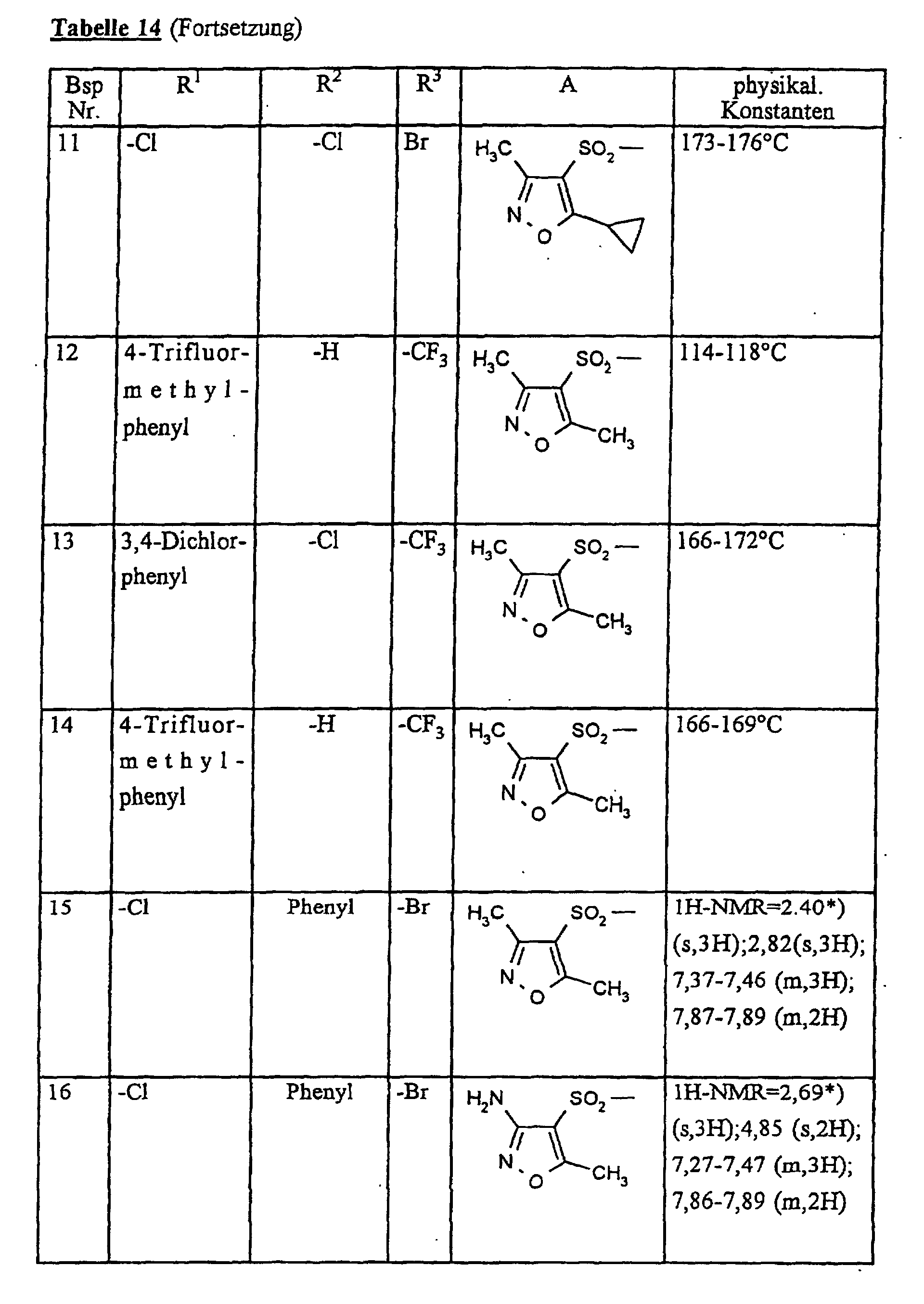

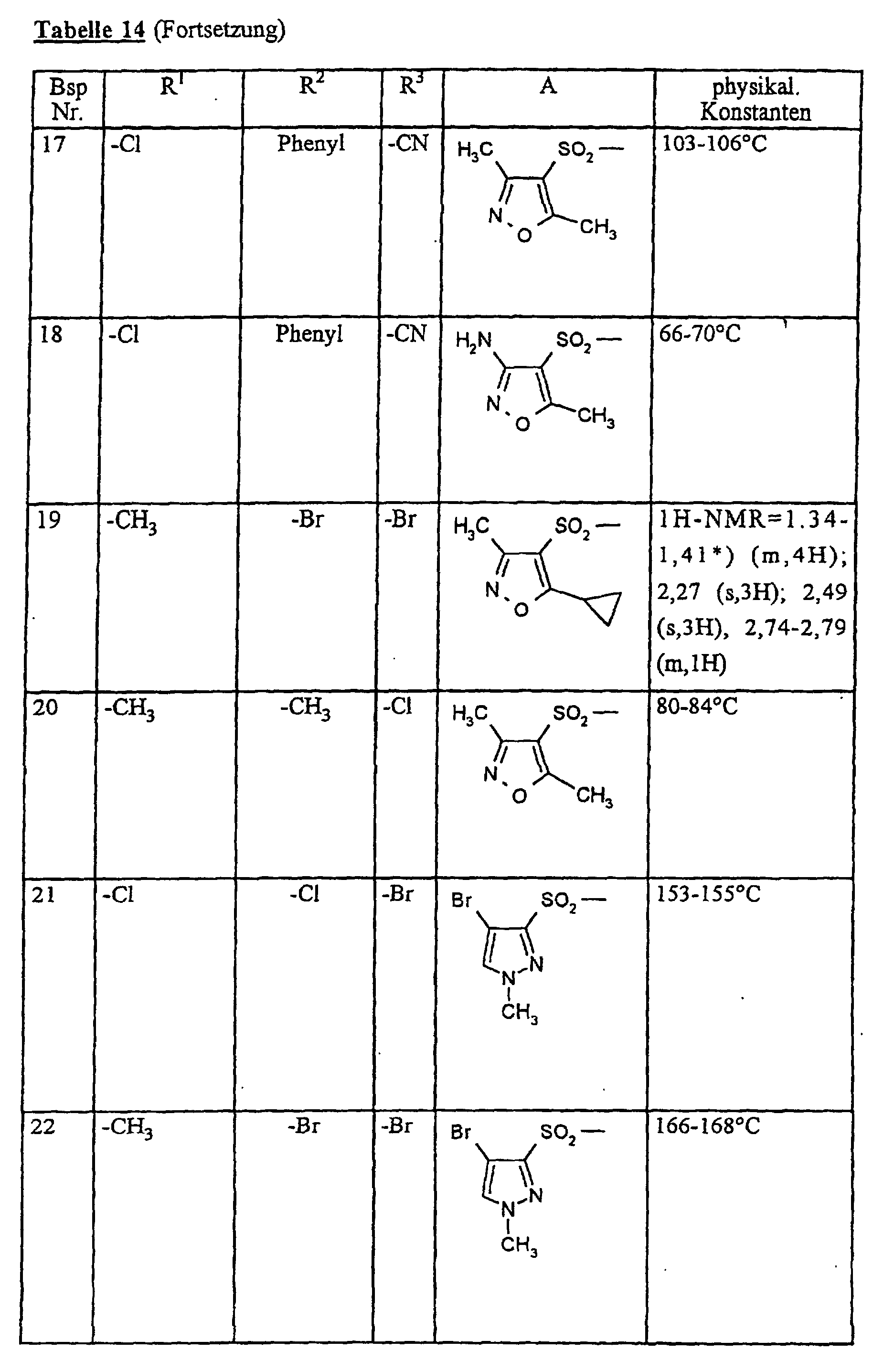

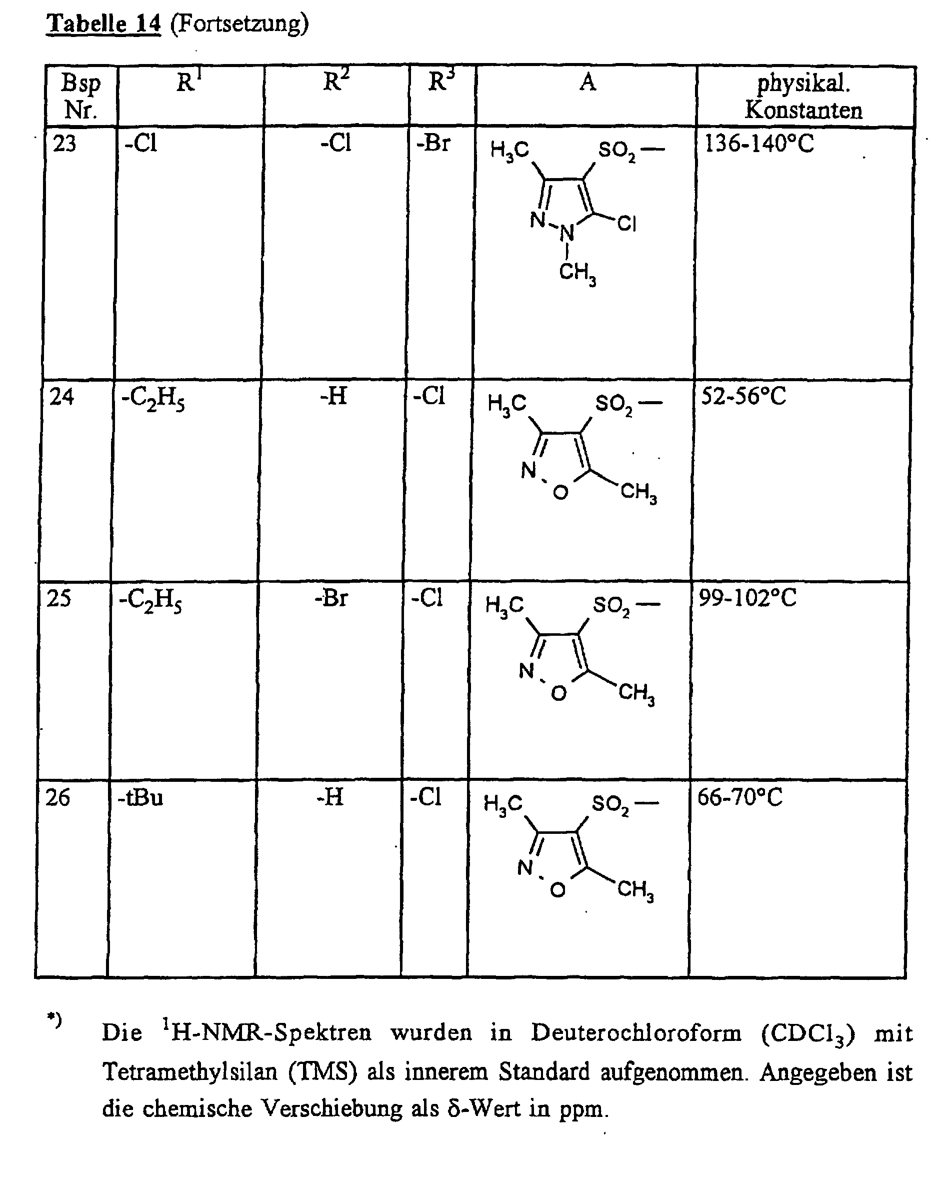

### Verwendungsbeispiele

### Beispiel A

### Phytophthora-Test (Tomate) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

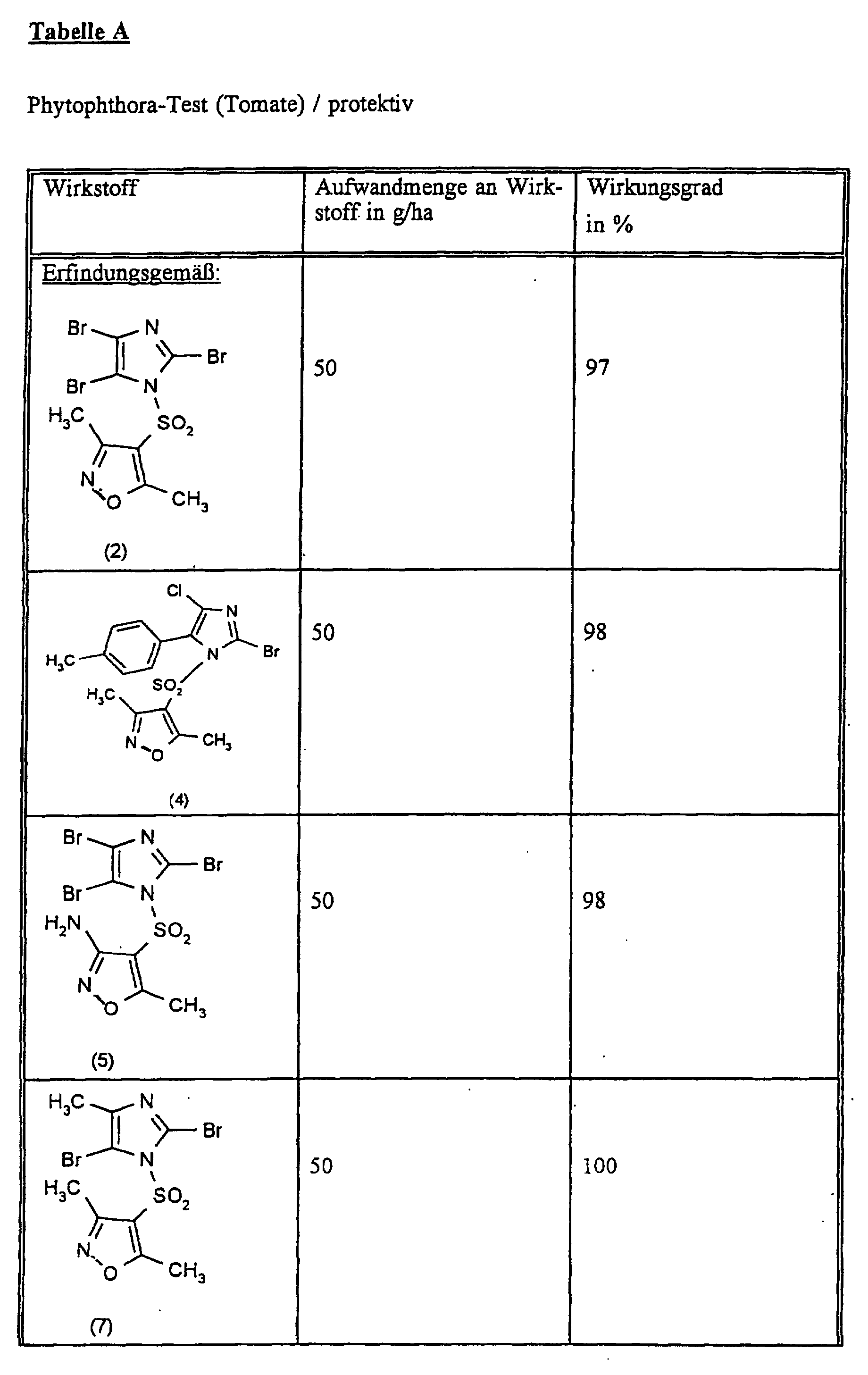

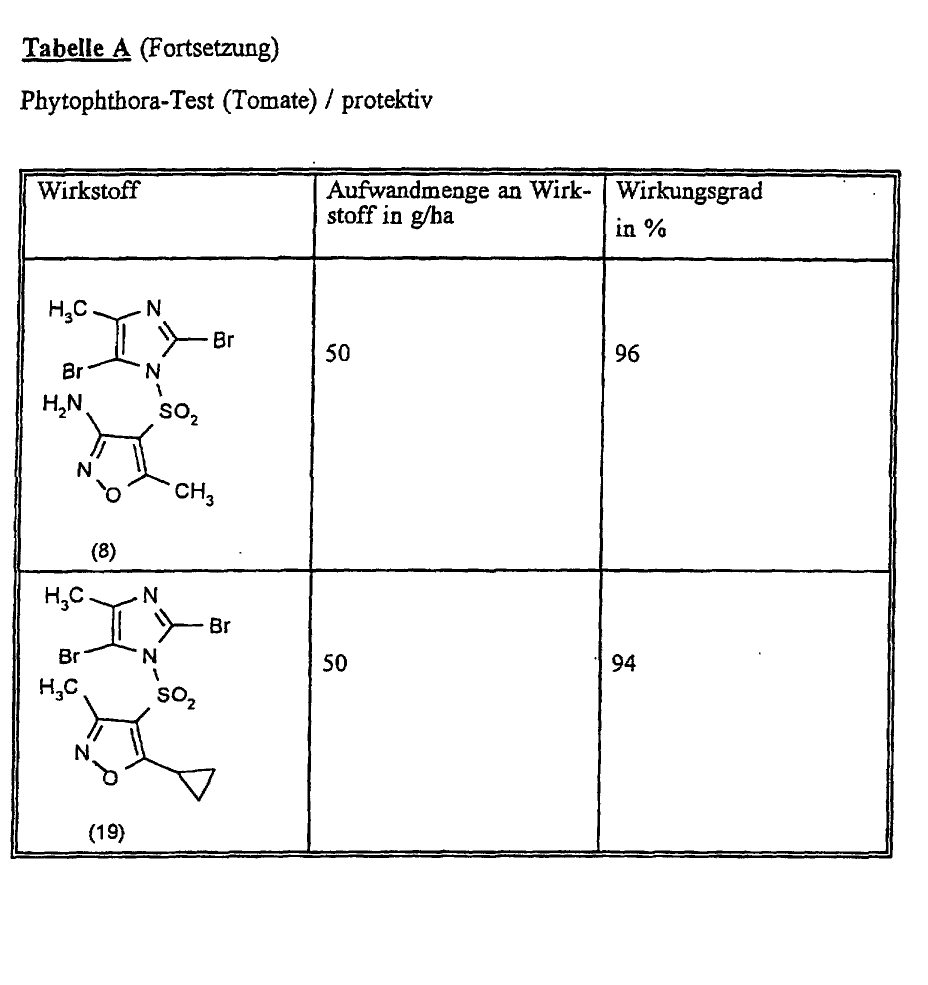

### Beispiel B

### Podosphaera-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70% aufgestellt.

10 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

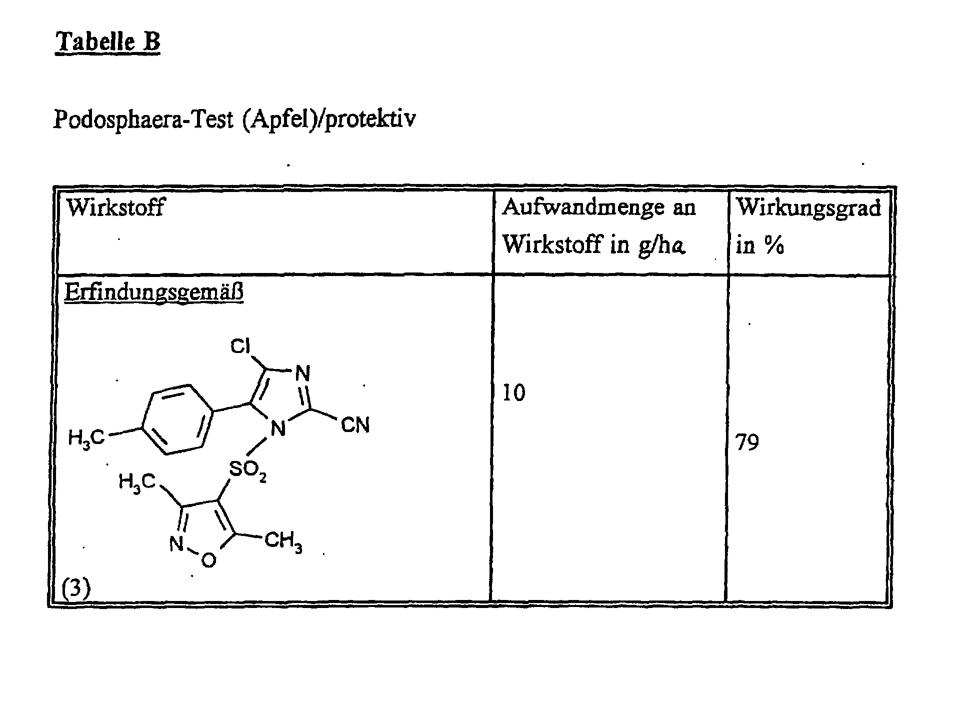

### Beispiel C

### Plasmopara-Test (Reben) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

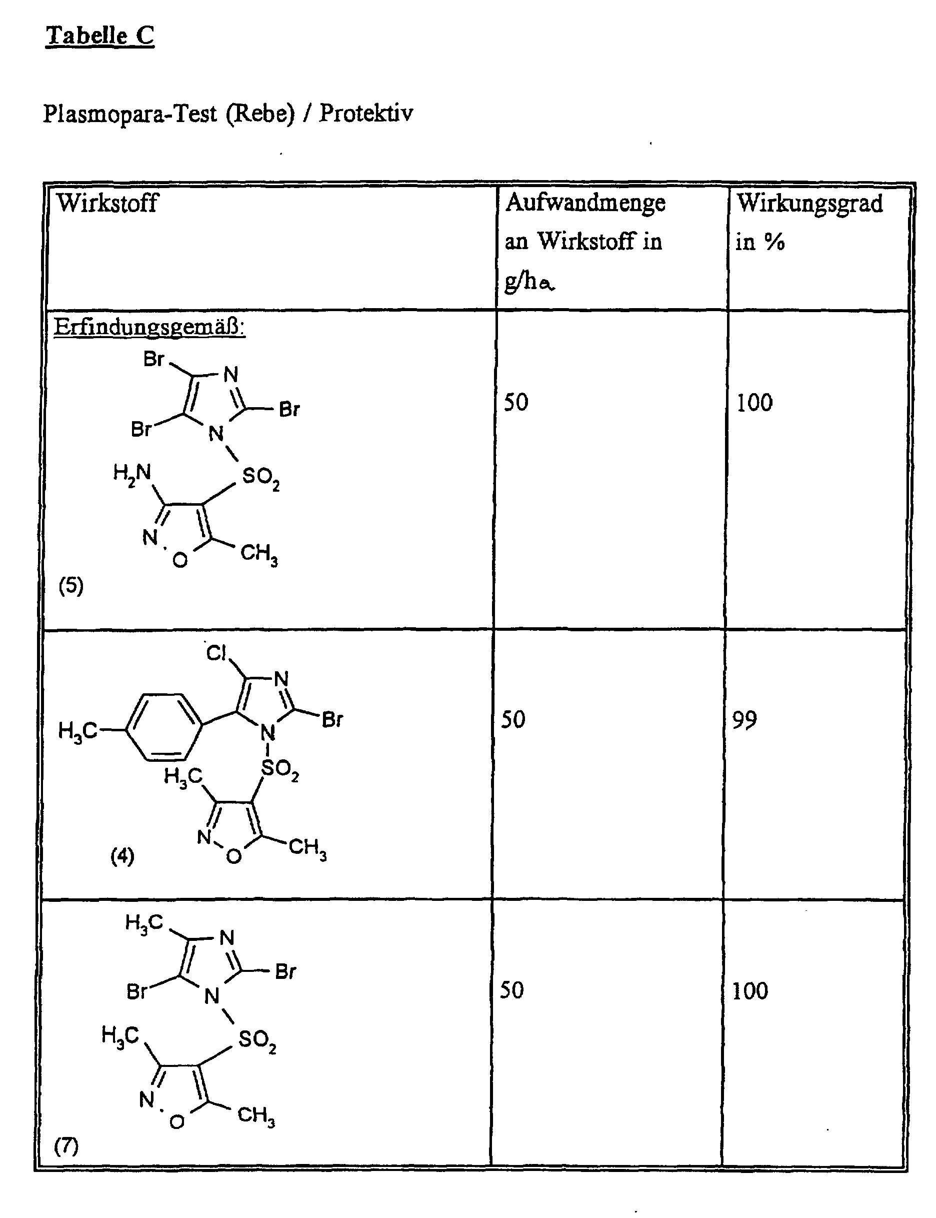

### Beispiel D

### Venturia-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine

Die Pflanzen werden dann im Gewächshaus bei 20°C und einer relativen Luftfeuchtigkeit von ca. 70% aufgestellt.

12 Tage nach der Inokulation erfolgt die Auswertung. Dabei bedeutet 0 % ein Wirkungsgrad, der demjenigen der unbehandelten Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

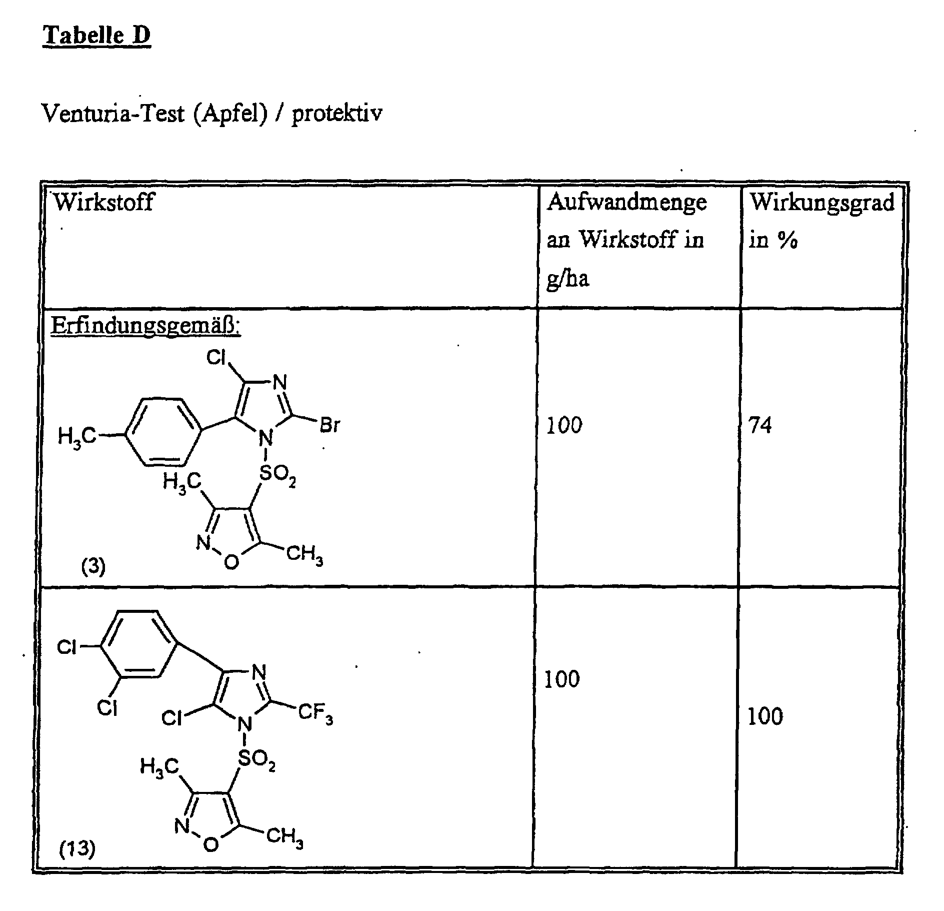

## Patentansprüche

1. Imidazolderivate der Formel 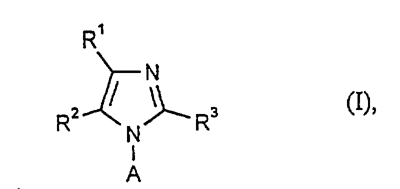 in welcher
R¹ und R² gleich oder verschieden sind und unabhängig voneinander für Wasserstoff, Fluor, Chlor, Brom, Iod, Cyano, Thiocyanato, Nitro, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil, Halogenalkylcarbonyloxy mit 1 bis 4 Kohlenstoffatomen in der Halogenalkylgruppe und 1 bis 5 gleichen oder verschiedenen Halogenatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Alkenyl mit 2 bis 4 Kohlenstoffatomen, Alkenyloxy mit 2 bis 4 Kohlenstoffatomen, Halogenalkylthio mit 1 bis 4 Kohlenstoffatomen und 1 bis 5 gleichen oder verschiedenen Halogenatomen, Halogenalkenyl mit 2 bis 4 Kohlenstoffatomen und 1 bis 5 gleichen oder verschiedenen Halogenatomen, Halogenalkenyloxy mit 2 bis 4 Kohlenstoffatomen und 1 bis 5 gleichen oder verschiedenen Halogenatomen, Alkylsulfonyloxy mit 1 bis 4 Kohlenstoffatomen oder für Z-R⁴ stehen, wobei
Z für Alkandiyl mit 1 bis 4 Kohlenstoffatomen, Alkendiyl mit 2 bis 4 Kohlenstoffatomen, Alkindiyl mit 2 bis 4 Kohlenstoffatomen oder eine Gruppe der Formel
-*Q-CQ-, -*CQ-Q-, -*CH₂-Q-, -*Q-CH₂-, -*CQ-Q-CH₂-,
-*CH₂-Q-CQ-, -*Q-CQ-CH₂-, -*Q-CQ-Q-CH₂-, -S(O)ₙ-,
-*CH₂-S(O)ₙ-, -CQ- oder -*S(O)ₙ-CH₂-
steht,
wobei jeweils die mit * gekennzeichneten Atome mit R⁴ verbunden sind,
Q für Sauerstoff oder Schwefel steht,
n für 0, 1 oder 2 steht,
und
R⁴ für Phenyl oder Naphthyl steht, wobei jeder dieser beiden Reste einfach bis dreifach, gleichartig oder verschieden substituiert sein kann durch Halogen, Cyano, Nitro, Formyl, Carbamoyl, Thiocarbamoyl, Alkyl mit 1 bis 4 Kohlenstoffatomen, Alkoxy mit 1 bis 4 Kohlenstoffatomen, Alkylthio mit 1 bis 4 Kohlenstoffatomen, Alkenyl oder Alkenyloxy mit jeweils 2 bis 4 Kohlenstoffatomen, Alkinyl oder Alkinyloxy mit jeweils 2 bis 4 Kohlenstoffatomen, Halogenalkyl, Halogenalkoxy oder Halogenalkylthio mit jeweils 1 bis 4 Kohlenstoffatomen und 1 bis 5 gleichen oder verschiedenen Halogenatomen, Halogenalkenyl oder Halogenalkenyloxy mit jeweils 2 bis 4 Kohlenstoffatomen und 1 bis 5 gleichen oder verschiedenen Halogenatomen, Halogenalkinyl oder Halogenalkinyloxy mit jeweils 2 bis 4 Kohlenstoffatomen und 1 bis 5 gleichen oder verschiedenen Halogenatomen, Alkylamino, Dialkylamino, Alkoxycarbonyl, Hydroximinoalkyl oder Alkoximinoalkyl mit jeweils 1 bis 4 Kohlenstoffatomen in den einzelnen Alkylteilen, Cycloalkyl mit 3 bis 6 Kohlenstoffatomen, Alkylsulfinyl mit 1 bis 4 Kohlenstoffatomen, Alkylsulfonyl mit 1 bis 4 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylcarbonyl mit 1 bis 4 Kohlenstoffatomen in der Alkylgruppe, Alkylcarbonyloxy mit 1 bis 4 Kohlenstoffatomen im Alkylteil und/oder Alkylsulfonyloxy mit 1 bis 4 Kohlenstoffatomen,
oder auch einfach substituiert sein kann durch gegebenenfalls einfach bis vierfach, gleichartig oder verschieden durch Halogen substituiertes, zweifach verknüpftes Dioxyalkylen mit 1 oder 2 Kohlenstoffatomen, wobei die Sauerstoffatome nicht benachbart sind,
oder
R⁴ für einen ungesättigten Heterocyclyl-Rest mit 5 oder 6 Ringgliedern und 1 bis 3 Heteroatomen, wie Stickstoff, Sauerstoff und/oder Schwefell steht, wobei diese Reste einfach bis dreifach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 3 Kohlenstoffatomen im Alkoxyteil, Cycloalkyl mit 3 bis 6 Kohlenstoffatomen, Cyano und/oder Nitro,
oder
Z für eine direkte Bindung oder für ein Sauerstoffatom steht, wenn
R⁴ für Phenyl oder Naphthyl steht, wobei jeder dieser beiden Reste einfach bis dreifach, gleichartig oder verschieden substituiert sein kann durch diejenigen Reste, die oben bereits als Phenyl- bzw. Naphthyl-Substituenten genannt wurden,
R³ für Fluor, Chlor, Brom, Iod, Cyano, Nitro, Trifluormethyl, Thiocarbamoyl, Thiocyanato oder die Gruppierung 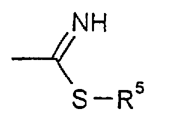 steht,
worin
R⁵ für Alkyl mit 1 bis 4 Kohlenstoffatomen steht,
A für eine Gruppierung der Formel -SO₂-R⁶ steht und
R⁶ für einen ungesättigten Heterocycyl-Rest mit 5 oder 6 Ringgliedern und 1 bis 3 Heteroatomen, wie Sauerstoff, Schwefel und/oder Stickstoff steht, wobei mindestens ein Heteroatom für Stickstoff oder Sauerstoff steht, und wobei die Heterocyclen einfach, zweifach oder auch dreifach gleichartig oder verschieden substituiert sein können durch Halogen, Cyano, Nitro, Hydroxy, Mercapto, Amino, Formyl, Carboxy, Carbamoyl, Thiocarbamoyl, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Alkoxycarbonyl mit 1 bis 4 Kohlenstoffatomen im Alkoxyteil, Cycloalkyl mit 3 bis 6 Kohlenstoffatomen, Halogenalkylsulfinyl mit 1 bis 4 Kohlenstoffatomen und 1 bis 5 gleichen oder verschiedenen Halogenatomen, Halogenalkylsulfonyl mit 1 bis 4 Kohlenstoffatomen und 1 bis 5 gleichen oder verschiedenen Halogenatomen, Alkylamino mit 1 bis 4 Kohlenstoffatomen, Hydroxyalkylamino mit 1 bis 4 Kohlenstoffatomen, Dialkylamino mit 1 bis 4 Kohlenstoffatomen in jeder Alkylgruppe, Alkylcarbonyl mit 1 bis 4 Kohlenstoffatomen im Alkylteil, Hydroxyiminoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylcarbonyloxy mit 1 bis 4 Kohlenstoffatomen im Alkylteil, Halogenalkylcarbonyloxy mit 1 bis 4 Kohlenstoffatomen in der Halogenalkylgruppe und 1 bis 5 gleichen oder verschiedenen Halogenatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Halogenalkylthio mit 1 bis 4 Kohlenstoffatomen und 1 bis 5 gleichen oder verschiedenen Halogenatomen, Alkylsulfonyloxy mit 1 bis 4 Kohlenstoffatomen, oder durch jeweils gegebenenfalls einfach oder zweifach durch Methyl substituiertes Pyrrolidinyl, Piperidinyl, Piperidyl, Morpholinyl oder Piperazinyl,
sowie deren Säureadditions-Salze und Metallsalz-Komplexe.

2. Verfahren zur Herstellung von Imidazolderivaten der Formel (I) gemäß Anspruch 1 sowie von deren Säureadditions-Salzen und Metallsalz-Komplexen, **dadurch gekennzeichnet, daß** man
a) Imidazole der Formel 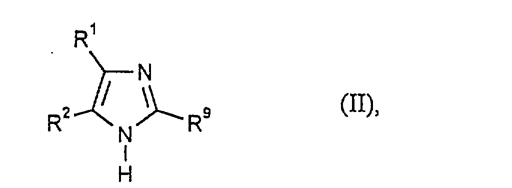 in welcher
R¹ und R² die oben angegebenen Bedeutungen haben und
R⁹ für Fluor, Chlor, Brom, Cyano, Nitro, Trifluormethyl oder Thiocyanato steht,
mit Halogeniden der Formel
A-X¹ (III)
in welcher
A die oben angegebene Bedeutung hat und
X¹ für Halogen steht,
gegebenenfalls in Gegenwart eines Säurebindemittels und gegebenenfalls in Gegenwart eines Verdünnungsmittels umsetzt,
oder wenn man
b) Cyanimidazole der Formel 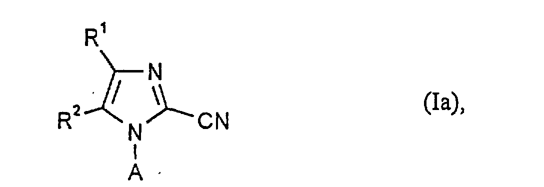 in welcher
R¹, R² und A die oben angegebenen Bedeutungen haben,
mit Schwefelwasserstoff, gegebenenfalls in Gegenwart einer Base und gegebenenfalls in Gegenwart eines Verdünnungsmittels umsetzt, oder wenn man
c) Thiocarbamoylimidazole der Formel 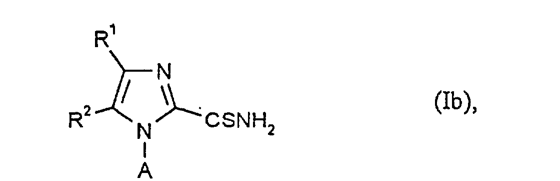 in welcher
R¹, R² und A die oben angegebenen Bedeutungen haben,
mit Alkylierungsmitteln der Formel
R⁵-X² (IV)
in welcher
R⁵ die oben angegebene Bedeutung hat und
X² für eine Abgangsgruppe steht,
gegebenenfalls in Gegenwart eines Säurebindemittels und gegebenenfalls in Gegenwart eines Verdünnungsmittels umsetzt,
und gegebenenfalls an die so erhaltenen Verbindungen der Formel (I) eine Säure oder ein Metallsalz addiert.

3. Mikrobizide Mittel, **gekennzeichnet durch** einen Gehalt an mindestens einem Imidazolderivat der Formel (I) gemäß Anspruch 1 bzw. an einem Säureadditonssalz oder Metallsalz-Komplex eines Imidazolderivates der Formel (I) neben Streckmitteln und/oder oberflächen aktiven Stoffen.

4. Verwendung von Imidazolderivaten der Formel (I) gemäß Anspruch 1 bzw. von deren Säureadditionssalzen oder Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, **dadurch gekennzeichnet, daß** man Imidazolderivate der Formel (I) gemäß Anspruch 1 bzw. deren Säureadditionssalze oder Metallsalz-Komplexe auf die Mikroorganismen und/oder deren Lebensaraum ausbringt.

6. Verfahren zur Herstellung von mikrobiziden Mitteln, **dadurch gekennzeichnet, daß** man Imidazolderivate der Formel (I) gemäß Anspruch 1 bzw. deren Säureadditionssalze oder Metallsalz-Komplexe mit Streckmitteln und/oder oberflächenaktiven Stoffen vermischt.

## Claims

1. Imidazole derivatives of the formula 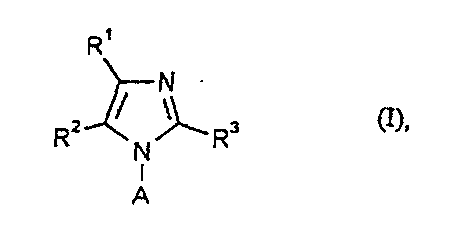 in which
R¹ and R² are identical or different and independently of one another represent hydrogen, fluorine, chlorine, bromine, iodine, cyano, thiocyanato, nitro, formyl, carboxyl, carbamoyl, thiocarbamoyl, alkyl having 1 to 4 carbon atoms, alkoxy having 1 to 4 carbon atoms, haloalkyl having 1 to 4 carbon atoms and 1 to 5 identical or different halogen atoms, haloalkoxy having 1 to 4 carbon atoms and 1 to 5 identical or different halogen atoms, alkoxycarbonyl having 1 to 4 carbon atoms in the alkoxy moiety, cycloalkyl having 3 to 6 carbon atoms, haloalkylsulfinyl having 1 to 4 carbon atoms and 1 to 5 identical or different halogen atoms, haloalkylsulfonyl having 1 to 4 carbon atoms and 1 to 5 identical or different halogen atoms, alkylamino having 1 to 4 carbon atoms, hydroxyalkylamino having 1 to 4 carbon atoms, dialkylamino having 1 to 4 carbon atoms in each alkyl group, alkylcarbonyl having 1 to 4 carbon atoms in the alkyl moiety, hydroxyiminoalkyl having 1 to 4 carbon atoms in the alkyl moiety, alkoxyiminoalkyl having 1 to 4 carbon atoms in the alkyl moiety and 1 to 4 carbon atoms in the alkoxy moiety, alkylcarbonyloxy having 1 to 4 carbon atoms in the alkyl moiety, haloalkylcarbonyloxy having 1 to 4 carbon atoms in the haloalkyl group and 1 to 5 identical or different halogen atoms, alkylthio having 1 to 4 carbon atoms, alkylsulfinyl having 1 to 4 carbon atoms, alkylsulfonyl having 1 to 4 carbon atoms, alkenyl having 2 to 4 carbon atoms, alkenyloxy having 2 to 4 carbon atoms, haloalkylthio having 1 to 4 carbon atoms and 1 to 5 identical or different halogen atoms, haloalkenyl having 2 to 4 carbon atoms and 1 to 5 identical or different halogen atoms, haloalkenyloxy having 2 to 4 carbon atoms and 1 to 5 identical or different halogen atoms, alkylsulfonyloxy having 1 to 4 carbon atoms or Z-R⁴, where
Z represents alkanediyl having 1 to 4 carbon atoms, alkenediyl having 2 to 4 carbon atoms, alkynediyl having 2 to 4 carbon atoms or a group of the formula
-*Q-CQ-, -*CQ-Q-, -*CH₂-Q-, -*Q-CH₂-, -*CQ-Q-CH₂-,
-*CH₂-Q-CQ-, -*Q-CQ-CH₂-, -*Q-CQ-Q-CH₂-, -S(O)ₙ-,
- *CH₂-S(O)ₙ-, -CQ- or -*S(O)ₙ-CH₂-,
where the atoms marked by * are in each case attached to R⁴,
Q represents oxygen or sulphur,
n represents 0, 1 or 2,
and
R⁴ represents phenyl or naphthyl, where each of these two radicals may be mono- to trisubstituted by identical or different substituents from the group consisting of halogen, cyano, nitro, formyl, carbamoyl, thiocarbamoyl, alkyl having 1 to 4 carbon atoms, alkoxy having 1 to 4 carbon atoms, alkylthio having 1 to 4 carbon atoms, alkenyl or alkenyloxy having in each case 2 to 4 carbon atoms, alkynyl or alkynyloxy having in each case 2 to 4 carbon atoms, haloalkyl, haloalkoxy or haloalkylthio having in each case 1 to 4 carbon atoms and 1 to 5 identical or different halogen atoms, haloalkenyl or haloalkenyloxy having in each case 2 to 4 carbon atoms and 1 to 5 identical or different halogen atoms, haloalkynyl or haloalkynyloxy having in each case 2 to 4 carbon atoms and 1 to 5 identical or different halogen atoms, alkylamino, dialkylamino, alkoxycarbonyl, hydroxyiminoalkyl or alkoxyiminoalkyl having in each case 1 to 4 carbon atoms in the individual alkyl moieties, cycloalkyl having 3 to 6 carbon atoms, alkylsulfinyl having 1 to 4 carbon atoms, alkylsulfonyl having 1 to 4 carbon atoms, haloalkylsulfinyl having 1 to 4 carbon atoms and 1 to 5 identical or different halogen atoms, haloalkylsulfonyl having 1 to 4 carbon atoms and 1 to 5 identical or different halogen atoms, alkylcarbonyl having 1 to 4 carbon atoms in the alkyl group, alkylcarbonyloxy having 1 to 4 carbon atoms in the alkyl moiety and alkylsulfonyloxy having 1 to 4 carbon atoms,
or else may be monosubstituted by doubly attached dioxyalkylene having 1 or 2 carbon atoms, which is optionally mono- to tetrasubstituted by identical or different halogen and where the oxygen atoms are not adjacent,
or
R⁴ represents an unsaturated heterocyclyl radical having 5 or 6 ring members and 1 to 3 heteroatoms, such as nitrogen, oxygen and/or sulphur, where these radicals may be mono- to trisubstituted by identical or different substituents from the group consisting of halogen, alkyl having 1 to 4 carbon atoms, alkoxy having 1 to 4 carbon atoms, haloalkyl having 1 to 4 carbon atoms and 1 to 5 identical or different halogen atoms, haloalkoxy having 1 to 4 carbon atoms and 1 to 5 identical or different halogen atoms, alkoxycarbonyl having 1 to 3 carbon atoms in the alkoxy moiety, cycloalkyl having 3 to 6 carbon atoms, cyano and nitro,
or
Z represents a direct bond or represents an oxygen atom when
R⁴ represents phenyl or naphthyl, where each of these two radicals may be mono- to trisubstituted by identical or different substituents from those radicals already mentioned above as phenyl or naphthyl substituents,
R³ represents fluorine, chlorine, bromine, iodine, cyano, nitro, trifluormethyl, thiocarbamoyl, thiocyanato or the grouping 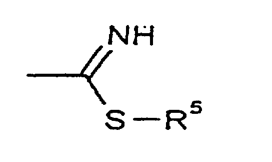 where
R⁵ represents alkyl having 1 to 4 carbon atoms,
A represents a grouping of the formula -SO₂-R⁶
and
R⁶ represents an unsaturated heterocyclyl radical having 5 or 6 ring members and 1 to 3 heteroatoms, such as oxygen, sulphur and/or nitrogen, where at least 1 heteroatom represents nitrogen or oxygen and where the heterocycles may be mono-, di- or else trisubstituted by identical or different substituents from the group consisting of halogen, cyano, nitro, hydroxyl, mercapto, amino, formyl, carboxyl, carbamoyl, thiocarbamoyl, alkyl having 1 to 4 carbon atoms, alkoxy having 1 to 4 carbon atoms, haloalkyl having 1 to 4 carbon atoms and 1 to 5 identical or different halogen atoms, haloalkoxy having 1 to 4 carbon atoms and 1 to 5 identical or different halogen atoms, alkoxycarbonyl having 1 to 4 carbon atoms in the alkoxy moiety, cycloalkyl having 3 to 6 carbon atoms, haloalkylsulfinyl having 1 to 4 carbon atoms and 1 to 5 identical or different halogen atoms, haloalkylsulfonyl having 1 to 4 carbon atoms and 1 to 5 identical or different halogen atoms, alkylamino having 1 to 4 carbon atoms, hydroxyalkylamino having 1 to 4 carbon atoms, dialkylamino having 1 to 4 carbon atoms in each alkyl group, alkylcarbonyl having 1 to 4 carbon atoms in the alkyl moiety, hydroxyiminoalkyl having 1 to 4 carbon atoms in the alkyl moiety, alkoxyiminoalkyl having 1 to 4 carbon atoms in the alkoxy moiety and 1 to 4 carbon atoms in the alkyl moiety, alkylcarbonyloxy having 1 to 4 carbon atoms in the alkyl moiety, haloalkylcarbonyloxy having 1 to 4 carbon atoms in the haloalkyl group and 1 to 5 identical or different halogen atoms, alkylthio having 1 to 4 carbon atoms, alkylsulfinyl having 1 to 4 carbon atoms, alkylsulfonyl having 1 to 4 carbon atoms, haloalkylthio having 1 to 4 carbon atoms and 1 to 5 identical or different halogen atoms, alkylsulfonyloxy having 1 to 4 carbon atoms, or else by pyrrolidinyl, piperidinyl, piperidyl, morpholinyl or piperazinyl, each of which is optionally mono- or disubstituted by methyl.
and their acid addition salts and metal salt complexes.

2. Process for preparing imidazole derivatives of the formula (I) according to Claim 1 and their acid addition salts and metal salt complexes, **characterized in that**
a) imidazoles of the formula 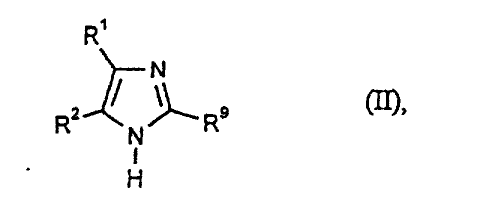 in which
R¹ and R² are as defined above
R⁹ represents fluorine, chlorine, bromine, cyano, nitro, trifluoromethyl or thiocyanato
are reacted with halides of the formula
A-X¹ (III)
in which
A is as defined above and
X¹ represents halogen,
if appropriate in the presence of an acid binder and if appropriate in the presence of a diluent,
or that
b) cyanoimidazoles of the formula 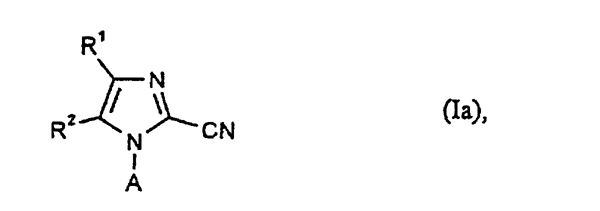 in which
R¹, R² and A are as defined above
or reacted with hydrogen sulphide, if appropriate in the presence of a base and if appropriate in the presence of a diluent, or that
c) thiocarbamoylimidazoles of the formula 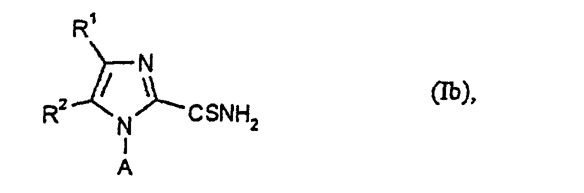 in which
R¹,R² and A are as defined above
are reacted with alkylating agents of the formula
R⁵-X² (IV)
in which
R⁵ is as defined above and
X² represents a leaving group,
if appropriate in the presence of an acid binder and if appropriate in the presence of a diluent,
and, if appropriate, an acid or a metal salt is added to the resulting compounds of the formula (I) .

3. Microbicidal compositions, **characterized in that** they comprise at least one imidazole derivative of the formula (I) according to Claim 1 or an acid addition salt or metal salt complex of an imidazole derivative of formula (I), in addition to extenders and/or surfactants.

4. Use of imidazole derivatives of the formula (I) according to Claim 1 or of their acid addition salts or metal salt complexes as microbicides in crop protection and in the protection of materials.

5. Method for controlling unwanted microorganisms in crop protection and in the protection of materials, **characterized in that** imidazole derivatives of the formula (I) according to Claim 1 or their acid addition salts or metal salt complexes are applied to the microorganisms and/or their habitat.

6. Process for preparing microbicidal compositions, **characterized in that** imidazole derivatives of the formula (I) according to Claim 1 or or their acid addition salts or metal salt complexes are mixed with extenders and/or surfactants.

## Revendications

1. Dérivés d'imidazole de formule 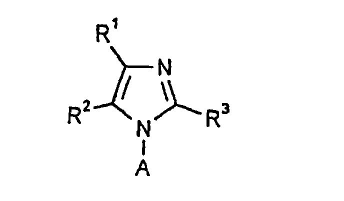 dans laquelle
R¹ et R² sont identiques ou différents et représentent, indépendamment l'un de l'autre, l'hydrogène, le fluor, le chlore, le brome, l'iode, un reste cyano, thiocyanato, nitro, formyle, carboxy, carbamoyle, thiocarbamoyle, alkyle ayant 1 à 4 atomes de carbone, alkoxy ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, alkoxycarbonyle ayant 1 à 4 atomes de carbone dans la partie alkoxy, cycloalkyle ayant 3 à 6 atomes de carbone, halogénalkylsulfinyle ayant 1 à 4 atomes de carbone et 1 à 5 atomes d'halogènes identiques ou différents, halogénalkylsulfonyle ayant 1 à 4 atomes de carbone et 1 à 5 atomes d'halogènes identiques ou différents, alkylamino ayant 1 à 4 atomes de carbone, hydroxyalkylamino ayant 1 à 4 atomes de carbone, dialkylamino ayant 1 à 4 atomes de carbone dans chaque groupe alkyle, alkylcarbonyle ayant 1 à 4 atomes de carbone dans la partie alkyle, hydroxyimino-alkyle ayant 1 à 4 atomes de carbone dans la partie alkyle, alkoximino-alkyle ayant 1 à 4 atomes de carbone dans la partie alkoxy et 1 à 4 atomes de carbone dans la partie alkyle, alkylcarbonyloxy ayant 1 à 4 atomes de carbone dans la partie alkyle, halogénalkylcarbonyloxy ayant 1 à 4 atomes de carbone dans le groupe halogénalkyle et 1 à 5 atomes d'halogènes identiques ou différents, alkylthio ayant 1 à 4 atomes de carbone, alkylsulfinyle ayant 1 à 4 atomes de carbone, alkylsulfonyle ayant 1 à 4 atomes de carbone, alcényle ayant 2 à 4 atomes de carbone, alcényloxy ayant 2 à 4 atomes de carbone, halogénalkylthio ayant 1 à 4 atomes de carbone et 1 à 5 atomes d'halogènes identiques ou différents, halogénalcényle ayant 2 à 4 atomes de carbone et 1 à 5 atomes d'halogènes identiques ou différents, halogénalcényloxy ayant 2 à 4 atomes de carbone et 1 à 5 atomes d'halogènes identiques ou différents, alkylsulfonyloxy ayant 1 à 4 atomes de carbone ou Z-R⁴,
où
Z est un reste alcanediyle ayant 1 à 4 atomes de carbone, alcènediyle ayant 2 à 4 atomes de carbone, alcynediyle ayant 2 à 4 atomes de carbone ou un groupe de formule
-*Q-CQ-, -*CQ-Q-, -*CH₂-Q-, -*Q-CH₂-, -*CQ-Q-CH₂-,
-*CH₂-Q-CQ-, -*Q-CQ-CH₂-, -*Q-CQ-Q-CH₂-, -S(O)ₙ-,
-*CH₂-S(O)ₙ-, -CQ- ou -*S(O)ₙ-CH₂-,
chacun des atomes de carbone identifiés par * étant lié à R⁴,
Q représente l'oxygène ou le soufre,
n a la valeur 0, 1 ou 2,
et
R⁴ est un reste phényle ou naphtyle, chacun de ces deux restes pouvant porter un à trois substituants, identiques ou différents, halogéno, cyano, nitro, formyle, carbamoyle, thiocarbamoyle, alkyle ayant 1 à 4 atomes de carbone, alkoxy ayant 1 à 4 atomes de carbone, alkylthio ayant 1 à 4 atomes de carbone, alcényle ou alcényloxy ayant chacun 2 à 4 atomes de carbone, alcynyle ou alcynyloxy ayant chacun 2 à 4 atomes de carbone, halogénalkyle, halogénalkoxy ou halogénalkylthio ayant chacun 1 à 4 atomes de carbone et 1 à 5 atomes d'halogènes identiques ou différents, halogénalcényle ou halogénalcényloxy ayant chacun 2 à 4 atomes de carbone et 1 à 5 atomes d'halogènes identiques ou différents, halogénalcynyle ou halogénalcynyloxy ayant chacun 2 à 4 atomes de carbone et 1 à 5 atomes d'halogènes identiques ou différents, alkylamino, dialkylamino, alkoxycarbonyle, hydroximinoalkyle ou alkoximino-alkyle ayant chacun 1 à 4 atomes de carbone dans les parties alkyle individuelles, cycloalkyle ayant 3 à 6 atomes de carbone, alkylsulfinyle ayant 1 à 4 atomes de carbone, alkylsulfonyle ayant 1 à 4 atomes de carbone, halogénalkylsulfinyle ayant 1 à 4 atomes de carbone et 1 à 5 atomes d'halogènes identiques ou différents, halogénalkylsulfonyle ayant 1 à 4 atomes de carbone et 1 à 5 atomes d'halogènes identiques ou différents, alkylcarbonyle ayant 1 à 4 atomes de carbone dans le groupe alkyle, alkylcarbonyloxy ayant 1 à 4 atomes de carbone dans la partie alkyle et/ou alkylsulfonyloxy ayant 1 à 4 atomes de carbone,
ou peut aussi porter un substituant dioxyalkylène à deux liaisons, ayant 1 ou 2 atomes de carbone, portant éventuellement un à quatre substituants halogéno identiques ou différents, et dont les atomes d'oxygène ne sont pas contigus,
ou bien
R⁴ est un reste hétérocyclyle non saturé à noyau de 5 ou 6 atomes ayant 1 à 3 hétéroatomes tels qu'azote, oxygène et/ou soufre, ces restes pouvant porter un à trois substituants, identiques ou différents, halogéno, alkyle ayant 1 à 4 atomes de carbone, alkoxy ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, alkoxycarbonyle ayant 1 à 3 atomes de carbone dans la partie alkoxy, cycloalkyle ayant 3 à 6 atomes de carbone, cyano et/ou nitro,
ou bien
Z représente une liaison directe ou un atome d'oxygène lorsque
R⁴ est un reste phényle ou naphtyle, chacun de ces deux restes pouvant être substitué une à trois fois identiques ou différentes par les restes qui ont déjà été mentionnés ci-dessus comme substituants de phényle ou de naphtyle,
R³ représente le fluor, le chlore, le brome, l'iode, un reste cyano, nitro, trifluorométhyle, thiocarbamoyle ou cyanato ou le groupement 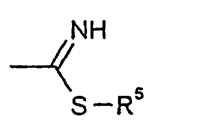 dans lequel
R⁵ est un reste alkyle ayant 1 à 4 atomes de carbone,
A représente un groupement de formule -SO₂-R⁶ et
R⁶ désigne un reste hétérocyclyle non saturé à noyau de 5 ou 6 atomes ayant 1 à 3 hétéroatomes tels qu'oxygène, soufre et/ou azote, au moins un hétéroatome représentant l'azote ou le soufre et les hétérocycles pouvant porter un, deux ou aussi trois substituants identiques ou différents, halogéno, cyano, nitro, hydroxy, mercapto, amino, formyle, carboxy, carbamoyle, thiocarbamoyle, alkyle ayant 1 à 4 atomes de carbone, alkoxy ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, alkoxycarbonyle ayant 1 à 4 atomes de carbone dans la partie alkoxy, cycloalkyle ayant 3 à 6 atomes de carbone, halogénalkylsulfinyle ayant 1 à 4 atomes de carbone et 1 à 5 atomes d'halogènes identiques ou différents, halogénalkylsulfonyle ayant 1 à 4 atomes de carbone et 1 à 5 atomes d'halogènes identiques ou différents, alkylamino ayant 1 à 4 atomes de carbone, hydroxyalkylamino ayant 1 à 4 atomes de carbone, dialkylamino ayant 1 à 4 atomes de carbone dans chaque groupe alkyle, alkylcarbonyle ayant 1 à 4 atomes de carbone dans la partie alkyle, hydroxyimino-alkyle ayant 1 à 4 atomes de carbone dans la partie alkyle, alkoximino-alkyle ayant 1 à 4 atomes de carbone dans la partie alkoxy et 1 à 4 atomes de carbone dans la partie alkyle, alkylcarbonyloxy ayant 1 à 4 atomes de carbone dans la partie alkyle, halogénalkylcarbonyloxy ayant 1 à 4 atomes de carbone dans le groupe halogénalkyle et 1 à 5 atomes d'halogènes identiques ou différents, alkylthio ayant 1 à 4 atomes de carbone, alkylsulfinyle ayant 1 à 4 atomes de carbone, alkylsulfonyle ayant 1 à 4 atomes de carbone, halogénalkylthio ayant 1 à 4 atomes de carbone et 1 à 5 atomes d'halogènes identiques ou différents, alkylsulfonyloxy ayant 1 à 4 atomes de carbone, ou pyrrolidinyle, pipéridinyle, pipéridyle, morpholinyle ou pipérazinyle portant chacun, le cas échéant, un ou deux substituants méthyle,
ainsi que leurs sels d'addition d'acides et leurs complexes de sels métalliques.

2. Procédé de production de dérivés d'imidazole de formule (I) suivant la revendication 1 ainsi que de leurs sels d'addition d'acides et de leurs complexes de sels métalliques, **caractérisé en ce que** :
a) on fait réagir des imidazoles de formule 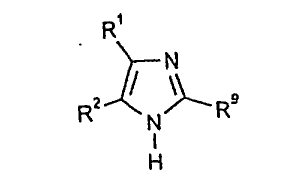 dans laquelle
R¹ et R² ont les définitions indiquées ci-dessus et
R⁹ représente le fluor, le chlore, le brome, un reste cyano, niro, trifluorométhyle ou thiocyanato,
avec des halogénures de formule
A-X¹ (III)
dans laquelle
A a la définition indiquée ci-dessus et
X¹ est un halogène,
le cas échéant en présence d'un accepteur d'acide et en présence éventuelle d'un diluant,
ou bien
b) on fait réagir des cyanimidazoles de formule 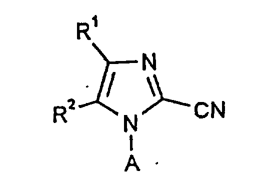 dans laquelle
R¹, R² et A ont les définitions indiquées ci-dessus,
avec le sulfure d'hydrogène, éventuellement en présence d'une base et en présence éventuelle d'un diluant,
ou bien
c) on fait réagir des thiocarbamoylimidazoles de formule 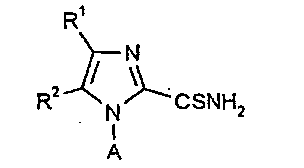 dans laquelle
R¹, R² et A ont les définitions indiquées ci-dessus,
avec des agents d'alkylation de formule
R⁵-X² (IV)
dans laquelle
R⁵ a la définition indiquée et
X² représente un groupe partant,
le cas échéant en présence d'un accepteur d'acide et en présence éventuelle d'un diluant,
et on additionne éventuellement un acide ou un sel métallique sur les composés de formule (I) ainsi obtenus.

3. Compositions microbicides, **caractérisées par** une teneur en au moins un dérivé d'imidazole de formule (I) suivant la revendication 1 ou en un sel d'addition d'acide ou un complexe de sel métallique d'un dérivé d'imidazole de formule (I), à côté de diluants et/ou d'agents tensioactifs.

4. Utilisation de dérivés d'imidazole de formule (I) suivant la revendication 1 ou de leurs sels d'addition d'acides ou de leurs complexes de sels métalliques comme microbicides dans la protection des plantes et dans la protection des matériaux.

5. Procédé pour combattre des micro-organismes indésirables dans la protection des plantes et dans la protection des matériaux, **caractérisé en ce qu'**on épand des dérivés d'imidazole de formule (I) suivant la revendication 1 ou leurs sels d'addition d'acides ou leurs complexes de sels métalliques sur les micro-organismes et/ou sur leur milieu.

6. Procédé de préparation de compositions microbicides, **caractérisé en ce qu'**on mélange des dérivés d'imidazole de formule (I) suivant la revendication 1 ou leurs sels d'addition d'acides ou leurs complexes de sels métalliques avec des diluants et/ou des agents tensioactifs.
